# EUROPÄISCHE PATENTANMELDUNG

(11) **EP 4 686 506 A1**
(43) Veröffentlichungstag der Anmeldung: **04.02.2026**
(21) Anmeldenummer: 24192130.3
(22) Anmeldetag: 31.07.2024
(51) Int. Cl.: B01L 3/00, F16K 99/00

(54) **MIKROFLUIDVORRICHTUNG UND MIKROFLUIDSYSTEM**

(71) Anmelder: ibidi GmbH, 82166 Gräfelfing (DE)
(72) Erfinder: Schwarz, Jan, 72511 Bingen (DE); Zantl, Roman, 82166 Gräfelfing (DE); Huber, Nina, 85221 Dachau (DE); Lubins, Georg, 80637 München (DE)
(74) Vertreter: Grünecker Patent- und Rechtsanwälte PartG mbB

(57) **Zusammenfassung**

Die vorliegende Erfindung betrifft eine Mikrofluidvorrichtung, umfassend ein Substrat, ein in dem Substrat angeordnetes Kanalsystem, einen ersten und einen zweiten fluidischen Anschluss für die Zuführung eines Fluids in das Kanalsystem, wobei die fluidischen Anschlüsse an einer ersten Seite des Substrats angeordnet sind, einen ersten und einen zweiten Ventilsitz, die beide in einer zweiten Seite des Substrats, die der ersten Seite gegenüberliegt, ausgebildet sind, und eine elastische Membran, die zumindest einen Teil der zweiten Seite des Substrats einschließlich der Ventilsitze bedeckt, wobei der erste Ventilsitz derart angeordnet ist, dass das Fluid vom ersten Anschluss durch den ersten Ventilsitz in das Kanalsystem strömen kann, und wobei der zweite Ventilsitz derart angeordnet ist, dass das Fluid vom zweiten Anschluss durch den zweiten Ventilsitz in das Kanalsystem strömen kann. Weiterhin betrifft die vorliegende Erfindung ein Mikrofluidsystem umfassend die Mikrofluidvorrichtung und eine Aktuatorvorrichtung.

## Beschreibung

Die vorliegende Erfindung betrifft eine Mikrofluidvorrichtung und ein Mikrofluidsystem umfassend die Mikrofluidvorrichtung.

In einer Vielzahl zellbiologischer Anwendungen werden Flüssigkeiten oder Gase (beide werden unter dem Begriff 'Fluid' zusammengefasst) durch ein Mikrofluidsystem geleitet, um damit Untersuchungen von Biofilmen oder Zellaggregaten vorzunehmen und das Verhalten von Zellen unter präzise steuerbaren Flussbedingungen zu untersuchen. Dazu können die Zellen in einer Beobachtungskammer auf einem Chip oder in einem Substrat vorliegen, und das Substrat wird mit dem Mikrofluidsystem verbunden, mit dem das Fluid durch die Beobachtungskammer geleitet wird. Dieser Aufbau findet beispielsweise Anwendung in der Arteriosklerose-Forschung im Zusammenhang mit dem Adhäsionsverhalten von Zellen, oder auch in der Simulation von Organmodellen, wobei physiologische Bedingungen wie in einem Organ (z.B. dem Darm) mit Hilfe des Mikrofluidsystems imitiert werden. Ein Mikrofluidsystem mit einer solchen Beobachtungskammer kann in einen Inkubator gestellt werden, sodass die Zellen darin kultiviert werden. Wünschenswert ist bei Mikrofluidsystemen eine hohe Flexibilität hinsichtlich der Anordnung verschiedener Systemkomponenten. Beispielweise soll es möglich sein, Fluide zuzuführen, ohne die bestehenden Flussbedingungen für die untersuchten Proben zu verändern.

Aus der EP 1 944 084 A1 ist ein Mikrofluidsystem bekannt, in dem eine Flüssigkeit zwischen zwei Reservoiren durch eine Beobachtungskammer geleitet wird. Unabhängig von der Pumprichtung fließt die Flüssigkeit dabei dank einer Gleichrichteranordnung von Ventilen stets in der gleichen Richtung durch die Beobachtungskammer.

Unter Umständen ist es nötig, der Flüssigkeit bedarfsweise weitere Komponenten beizugeben, beispielsweise Nährlösungen, Enzyme, Botenstoffe, Medikamente etc. Dabei soll allerdings der bestehende Fluss nicht gestört werden, weil dies unerwünschte Auswirkungen auf die untersuchten Zellen haben könnte. Für diese Problematik stellt der Stand der Technik bisher keine zufriedenstellende Lösung bereit.

Es ist daher eine Aufgabe der vorliegenden Erfindung, eine Mikrofluidvorrichtung bereitzustellen, in der verschiedene Komponenten einem Fluidfluss in der Mikrofluidvorrichtung beigeben zu können, ohne dabei die bestehenden Kultivierungsbedingungen für die Zellen unter einem Fluidfluss in der Mikrofluidvorrichtung oder einem Mikrofluidsystem zu beeinträchtigen.

Diese Aufgabe wird durch die Mikrofluidvorrichtung nach Anspruch 1 gelöst. Weitere Aspekte finden sich in den zugehörigen Unteransprüchen.

Erfindungsgemäß wird eine Mikrofluidvorrichtung bereitgestellt, die ein Substrat, ein in dem Substrat angeordnetes Kanalsystem, einen ersten und einen zweiten fluidischen Anschluss für die Zuführung eines Fluids in das Kanalsystem, wobei die fluidischen Anschlüsse an einer ersten Seite des Substrats angeordnet sind, einen ersten und einen zweiten Ventilsitz, die beide in einer zweiten Seite des Substrats, die der ersten Seite gegenüberliegt, ausgebildet sind, und eine elastische Membran, die zumindest einen Teil der zweiten Seite des Substrats einschließlich der Ventilsitze bedeckt, umfasst. Dabei ist der erste Ventilsitz derart angeordnet, dass das Fluid vom ersten Anschluss durch den ersten Ventilsitz in das Kanalsystem strömen kann, und der zweite Ventilsitz ist derart angeordnet, dass das Fluid vom zweiten Anschluss durch den zweiten Ventilsitz in das Kanalsystem strömen kann.

Mit einem oder mehreren der fluidischen Anschlüsse können Fluidreservoire verbunden sein, sodass bei geöffneten Ventilen das Fluid aus dem jeweiligen Fluidreservoir in das Kanalsystem strömt. Durch gezieltes Öffnen und Schließen der Ventilsitze wird gesteuert, welche Fluide in welcher Menge in das Kanalsystem fließen. Es kann damit ein präzises Mischungsverhältnis von Fluiden in dem Kanalsystem hergestellt werden. Weiterhin kann das Kanalsystem mit einer Beobachtungskammer verbunden sein, in der sich eine mikrobiologische Probe befindet. Diese Probe wird mit dem Fluidgemisch aus dem Kanalsystem versorgt. Handelt es sich bei den Fluiden beispielsweise um (flüssige) Nährlösungen, kann eine präzise Versorgung der Probe mit Nährstoffen erfolgen. Insofern kann das Mikrofluidsystem für die Bereitstellung einer präzise abgestimmten Nährlösung für mikrobiologische Proben sorgen. Erfolgt die Zugabe von Fluiden in das Kanalsystem zudem ausschließlich durch die Ventilsitze, werden die Strömungsverhältnisse im Kanalsystem der Mikrofluidvorrichtung und einer angeschlossenen Beobachtungskammer nicht beeinflusst.

Das Fluid kann eine Flüssigkeit und/oder ein Gas sein.

Das Substrat kann einen Kunststoff umfassen oder aus diesem bestehen. Insbesondere kann es Kunststoffe wie COC (Cyclo-Olefin-Copolymer), COP (Cyclo-Olefin-Polymer), PC (Polycarbonat), PS (Polystyrol), PE (Polyethylen), PMMA (Polymethylmetacrylat) oder einen transparenten Thermoplast oder ein Elastomer umfassen bzw. aus einem dieser Kunststoffe oder einer Mischung daraus bestehen.

Das Substrat kann ein Spritzgussteil sein, also durch Spritzguss hergestellt worden sein. Durch die Verwendung der genannten Materialien und Verfahren kann eine Mikrofluidvorrichtung kostengünstig und in großer Stückzahl und konstanter Qualität produziert werden.

Alternativ kann das Substrat ein Glas umfassen. Das Glas oder der Kunststoff können insbesondere die Doppelbrechung und die Autofluoreszenz eines Schott-Deckglases (wie D 263 M Schott Glas, Nr. 1.5H (170 +/- 10 µm)) aufweisen.

Das Substrat kann transparent sein, vor allem im sichtbaren Wellenlängenbereich. Dadurch kann die zugehörige Mikrofluidvorrichtung für optische Untersuchungen verwendet werden, beispielsweise (inverse) Mikroskopie.

Ein derart optisch hochwertiges Material kann Mikroskopieuntersuchungen mit hoher Präzision und geringen optischen Imperfektionen ermöglichen.

Das Substrat und die fluidischen Anschlüsse können aus dem gleichen Material bestehen. Ebenso können die fluidischen Anschlüsse Teil des Substrats sein. Weiterhin kann das Substrat mit den fluidischen Anschlüssen als ein Stück gefertigt sein. Dadurch wird die Herstellbarkeit der Mikrofluidvorrichtung weiter vereinfacht.

Das in dem Substrat ausgebildete Kanalsystem verbindet die fluidischen Anschlüsse der Mikrofluidvorrichtung miteinander. Das Kanalsystem kann einen Kanal oder eine Mehrzahl an Kanälen umfassen. Bei einer Mehrzahl an Kanälen können diese untereinander verbunden sein und ein Netzwerk bilden. Ein Kanal kann ein im Substrat ausgebildeter Hohlraum oder ein im Substrat ausgebildeter Graben, der durch ein weiteres Element, beispielsweise eine Folie, abdeckt ist, sein.

Die Ventilsitze sind derart angeordnet, dass ein Fluid von einem der fluidischen Anschlüsse durch den Ventilsitz strömen muss, um in das Kanalsystem zu gelangen. Es kann also gesteuert werden, ob und wann der Zufluss zum Kanalsystem von den Anschlüssen freigegeben wird. Wenn sich im Kanalsystem bereits ein Fluid befindet, kann die Zugabe weiterer Stoffe von außen gesteuert werden, ohne einen bestehenden Fluss im Kanalsystem unterbrechen zu müssen. Damit bleiben die Kultivierungsbedingungen für Zellen, die dem Fluss im Kanalsystem oder in einer mit dem Kanalsystem verbundenen Beobachtungskammer ausgesetzt sind, im Wesentlichen unverändert und werden nur durch die hinzugegebenen Stoffe in gewünschter Weise beeinflusst.

Ein Ventilsitz bezeichnet den Teil eines Ventils, der im geöffneten Zustand des zugehörigen Ventils von dem Fluid durchströmt wird, wobei die Strömung vom fluidischen Anschluss in das Kanalsystem oder in umgekehrter Richtung erfolgt. Entsprechend wird in einem geschlossenen Zustand des zugehörigen Ventils die Strömung blockiert. Der geschlossene Zustand kann erreicht werden, indem die Membran in den Ventilsitz gedrückt wird.

An der ersten Seite sind der erste und der zweite fluidische Anschluss angeordnet. Dabei kann die erste Seite im Wesentlichen plan ausgebildet sein. Von der planen ersten Seite können die Anschlüsse hervorragen oder in das Substrat hineinragen.

Die zweite Seite des Substrats kann plan ausgebildet sein. Alternativ kann die zweite Seite eine Vertiefung aufweisen, in der die Membran angeordnet ist. Dabei kann die Vertiefung die gleiche Dicke bzw. Tiefe wie die Membran aufweisen, sodass die Membran und die zweite Seite fluchtend ausgebildet sind. Es besteht also ein bündiger Abschluss der Membran mit der zweiten Seite. Eine Unterseite der Mikrofluidvorrichtung, also die zweite Seite des Substrats mit der aufgebrachten Membran kann in diesem Fall plan sein.

Die Mikrofluidvorrichtung ist insbesondere für Anwendungen mit (inverser) Mikroskopie ausgebildet, bei denen das Substrat bzw. die Mikrofluidvorrichtung (mit der Membran) als Objektträger fungiert. Eine plane Oberfläche verringert optische Imperfektionen und verbessert die Stabilität der Mikrofluidvorrichtung auf einem Objekttisch des Mikroskops.

Das Kanalsystem kann zumindest teilweise in Form eines Grabens an der zweiten Seite des Substrats ausgebildet sein. Die Membran kann den Graben fluidisch abdichten. Alternativ kann eine Folie, insbesondere eine Klebefolie, den Graben abdecken. Der Graben kann somit an einer Seite von der Membran oder der Folie und an den übrigen Seiten von dem Substrat begrenzt sein. Das Kanalsystem kann einen oder mehrere Kanäle umfassen. Es können alle Kanäle oder einzelne Kanäle in Form eines Grabens ausgebildet sein.

Die Struktur eines Grabens in der Oberfläche des Substrats kann einfach mittels Spritzguss erzeugt werden.

Das Kanalsystem kann derart mit den Ventilsitzen verbunden sein, dass das Fluid vom Ventilsitz in das Kanalsystem bzw. vom Kanalsystem in den Ventilsitz strömen kann, wenn der Ventilsitz im geöffneten Zustand ist. Mit anderen Worten, es kann eine fluidische Verbindung zwischen dem Kanalsystem und den Ventilsitzen bestehen, wobei diese Verbindung nur dann für einen Fluidfluss geöffnet ist, wenn die Membran nicht aktiv in den Ventilsitz gedrückt ist.

Der Ventilsitz kann eine Mündung zwischen einem Kanalsystem und einem Durchgangsloch durch das Substrat von der ersten Seite zur zweiten Seite sein. Von einem Anschluss auf der ersten Seite des Substrats kann das Fluid durch das Durchgangsloch hindurch zur zweiten Seite des Substrats strömen, wo eine Verbindung zum Kanalsystem besteht. Diese Verbindung kann blockiert werden, wenn die Membran in oder auf die Mündung des Durchgangslochs gedrückt wird. Der Ventilsitz ist dann geschlossen und es gelangt kein Fluid vom Anschluss in das Kanalsystem.

Die Membran kann eine Silikonmembran sein. Die Silikonmembran kann Elastosil umfassen oder daraus bestehen. Ebenso kann die Membran ein thermoplastisches Elastomer wie Flexdym umfassen oder daraus bestehen. Alternativ kann die Membran auch ein COC umfassen oder daraus bestehen.

Durch die Wahl dieser Materialien ist die Membran dazu geeignet, eine Vielzahl an Schaltzyklen zu überstehen, ohne dass ihrer Elastizität oder ihre Fähigkeit des Ventilschlusses beeinträchtigt wird.

Weiterhin kann die Membran mittels Spitzguss hergestellt worden sein. Insbesondere kann die Membran ein elastisches Silikon-Spritzgussteil sein. Dadurch erlangt man sowohl die Vorteile des Spritzgussverfahrens, als auch die Vorteile von Silikon als Material für die Membran.

Die Membran kann mehrere Schichten umfassen. Beispielsweise kann eine erste Schicht Ausbeulen durch Ermüdung der Elastizität verringern. Eine zweite Schicht kann eine hohe Elastizität aufweisen, um die Dichteigenschaften gegenüber dem Ventilsitz zu verbessern.

Die Membran kann eine Dicke im Bereich zwischen 5 µm und 5 mm, insbesondere zwischen 50 µm und 250 µm, aufweisen.

Die Dicke der Membran ist so gewählt, dass die Membran eine hohe Anzahl an Schaltzyklen bei konstanter Elastizität übersteht. Eine zu dünne Membran könnte zum Beispiel unter Druck reißen oder bereits nach kurzer Verwendungsdauer ihre Elastizität verlieren. Eine zu dicke Membran hingegen würde einen hohen Kraftaufwand benötigen, um sie in den Ventilsitz zu drücken.

Die Membran kann transparent sein, insbesondere im sichtbaren Wellenlängenbereich. Dies erlaubt optische Untersuchungen, beispielsweise mit einem (inversen) Mikroskop durch die Membran hindurch.

Zwischen dem Substrat und der Membran kann weiterhin eine doppelseitige Klebefolie angeordnet sein, und wobei die Membran mittels der Klebefolie an dem Substrat angebracht ist. Die Klebefolie kann die Ventilsitze aussparen. Die Membran kann zudem auch den Graben aussparen.

Die Anbringung bzw. Befestigung der Membran am Substrat mit Hilfe einer Klebefolie stellt eine einfache und effektive Methode der Anbringung dar.

In diesem Fall kann die Vertiefung in der zweiten Seite des Substrats eine Dicke bzw. Tiefe aufweisen, die der Summe der Dicken der Membran und der Klebefolie entspricht. So kann bei der Befestigung der Membran mit einer Klebefolie ein bündiger Abschluss mit der zweiten Seite des Substrats erreicht werden. Die Unterseite der Mikrofluidvorrichtung ist damit plan ausgebildet.

Alternativ kann die Membran durch Ultraschallschweißen oder Lösungsmittelschweißen, insbesondere auch unter Nutzung von Verkrallungseffekten in dafür vorgesehenen Öffnungen oder Rauigkeiten, an dem Substrat angebracht werden. Denkbar ist auch eine Klemmverbindung, bei der die Membran mittels einer Dichtlippe auf das Substrat geklemmt wird. Klebstoff oder dergleichen wird bei dieser Methode nicht benötigt.

Die Mikrofluidvorrichtung kann weiterhin eine Beobachtungskammer umfassen, die mit dem Kanalsystem über einen Zulauf und einen Ablauf fluidisch verbunden ist. Insbesondere kann die Beobachtungskammer in dem Substrat ausgebildet sein. Der Zulauf und der Ablauf können Mündungen am Kanalsystem sein. Alternativ können der Zulauf und der Ablauf Verbindungsschläuche zwischen dem Kanalsystem und der Beobachtungskammer sein. Der Zulauf und der Ablauf können eigene Kanäle sein, die nicht Teil des Kanalsystems sind.

Die Beobachtungskammer kann der Aufnahme und Kultivierung von Zellen, Zellaggregaten oder anderer zu untersuchender biologischer Proben dienen. Dort werden die Zellen auch unter bestimmten Umgebungsbedingungen kontrolliert, was die Versorgung mit Nährstoffen einschließt. Die Beobachtungskammer ist mit dem Kanalsystem über einen Zulauf und einen Ablauf verbunden, sodass eine Flüssigkeit aus dem Kanalsystem auch der Beobachtungskammer zugeführt wird. Insbesondere führt ein Fluss der Flüssigkeit im Kanalsystem auch zu einem Fluss in der Beobachtungskammer. Durch diesen Fluss können besagte Zellen in der Beobachtungskammer kultiviert werden, indem beispielsweise über die Flüssigkeit Nährstoffe zugeführt werden.

Falls die Beobachtungskammer in dem Substrat ausgebildet ist, kann das Substrat nur im Bereich der Beobachtungskammer transparent sein, insbesondere im sichtbaren Wellenlängenbereich. In den übrigen Bereichen kann das Substrat hingegen opak oder transluzent sein. Mikroskopische Beobachtungen werden in der Regel nur im Bereich der Beobachtungskammer durchgeführt, während die übrigen Bereiche des Substrats für solche Beobachtungen nur eine geringe Relevanz haben. Insofern bestehen für die übrigen Bereiche wesentlich geringere Anforderungen an die optische Qualität, sodass kostengünstigere Materialien eingesetzt werden können.

Zwischen dem Zulauf der Beobachtungskammer und dem Kanalsystem, sowie zwischen dem Ablauf der Beobachtungskammer und dem Kanalsystem kann jeweils ein weiterer Ventilsitz angeordnet sein. Die weiteren Ventilsitze können im Substrat ausgebildet sein.

Diese zusätzlichen Ventilsitze geben die Möglichkeit, die Beobachtungskammer von dem Kanalsystem abzusperren, wenn die Ventilsitze mit der Membran verschlossen werden. Dies kann nützlich sein, wenn beispielsweise ein Fluidaustausch (dies schließt die Möglichkeit des Austauschs einer Flüssigkeit durch ein Gas oder umgekehrt ein) in dem Kanalsystem vorgenommen werden soll, aber die Zellen (zunächst) dadurch nicht beeinflusst werden sollen. Ein anderer Fall ist eine mögliche Störung des Fluidflusses im Kanalsystem, zum Beispiel durch zu hohe unerwünschte Flüsse oder das Eindringen von Luftblasen. Hier können die Zellen in der Beobachtungskammer dadurch geschützt werden, dass die Ventilsitze geschlossen werden. Insgesamt ermöglichen die beiden zusätzlichen Ventilsitze also einen effizienten Schutz biologischer Proben in der Beobachtungskammer vor möglichweise schädlichen äußeren Einflüssen.

Das Kanalsystem kann weiterhin einen Bypass umfassen, sodass die Flüssigkeit durch das Kanalsystem an der Beobachtungskammer vorbeiströmen kann.

Diese Anordnung mit einem zusätzlichen Bypass erlaubt einen effizienten Fluidaustausch im Kanalsystem, wobei die Beobachtungskammer von diesem Fluidaustausch zunächst nicht betroffen ist. Das Fluid kann dazu zwischen dem ersten Anschluss und dem zweiten Anschluss über den Bypass aus dem Kanalsystem gespült werden.

Die Mikrofluidvorrichtung kann weiterhin ein erstes Flüssigkeitsreservoir und ein zweites Flüssigkeitsreservoir umfassen, wobei das erste Flüssigkeitsreservoir mit dem ersten fluidischen Anschluss verbunden ist, und wobei das zweite Flüssigkeitsreservoir mit dem zweiten fluidischen Anschluss verbunden ist. Insbesondere können das erste Flüssigkeitsreservoir und/oder das zweite Flüssigkeitsreservoir direkt auf den jeweiligen Anschluss gesteckt sein. Alternativ können das erste Flüssigkeitsreservoir und/oder das zweite Flüssigkeitsreservoir im dem Substrat ausgebildet sein, insbesondere als Hohlraum im Substrat.

Die Flüssigkeitsreservoire dienen der Bereitstellung eines Fluids für mikrobiologische Untersuchungen. Das Fluid kann insbesondere vom ersten Flüssigkeitsreservoir durch das Kanalsystem in das zweite Flüssigkeitsreservoir oder in umgekehrter Richtung förderbar sein.

Durch das direkte Aufstecken der Flüssigkeitsreservoire auf die Anschlüsse entfallen etwaige Schlauchverbindungen, sodass die Komplexität des gesamten Aufbaus deutlich reduziert werden kann.

Die fluidischen Anschlüsse können konisch ausgebildet sein. Insbesondere können die Anschlüsse dem Luer-Standard entsprechen. Die fluidischen Anschlüsse der Mikrofluidvorrichtung einen männlichen oder weiblichen Luer- oder Luerlock-Adapter aufweisen. Im bestimmungsgemäßen Gebrauch wird ein Schlauch oder ein Flüssigkeitsreservoir auf den jeweiligen Anschluss gesteckt. Durch die Verwendung konischer Anschlüsse, insbesondere von Anschlüssen, die dem Luer-Standard entsprechen, können die Anschlüsse beim Befüllen flüssigkeitsdicht verbunden werden und das Befüllen dadurch einfach und verlässlich durchgeführt werden. Mit dem Luer-Standard ist die Mikrofluidvorrichtung mit einer Vielzahl von Schläuchen und Reservoiren, die für den Anschluss vorgesehen sind, kompatibel. Weiterhin ist das direkte Aufstecken von Flüssigkeitsreservoiren auf weibliche Luer- oder Luerlock-Adapter besonders einfach.

Alternativ können die fluidischen Anschlüsse als Barbed Tee-Anschlüsse ausgebildet sein. Denkbar ist auch, dass manche fluidischen Anschlüsse der Mikrofluidvorrichtung konisch ausgebildet sind, während andere als Barbed Tee-Anschlüsse ausgebildet sind.

Weiterhin kann die Mikrofluidvorrichtung einen dritten fluidischen Anschluss und einen vierten fluidischen Anschluss, sowie ein drittes und ein viertes Flüssigkeitsreservoir, das mit dem dritten bzw. vierten fluidischen Anschluss verbunden ist, umfassen, wobei der dritte und der vierte Anschluss mit dem Kanalsystem fluidisch verbunden sind.

Diese Anordnung mit vier Flüssigkeitsreservoiren erhöht die Flexibilität der Mikrofluidvorrichtung, weil eine Mehrzahl an Flüssigkeiten in das Kanalsystem kontrolliert durch Schalten der entsprechenden Ventile (bzw. Ventilsitze) eingeleitet werden kann. Dies ermöglicht das Studium von Zellen in der Beobachtungskammer unter dem Einfluss mehrerer (unterschiedlicher) Flüssigkeiten.

Auch in diesem Fall gilt, dass alle Flüssigkeitsreservoire direkt auf den jeweiligen Anschluss gesteckt sein können.

Es ist denkbar zwei der beschriebenen Mikrofluidvorrichtungen in einer Kaskade anzuordnen. Dabei wird ein fluidischer Anschluss der ersten Mikrofluidvorrichtung mit einem fluidischen Anschluss der zweiten Mikrofluidvorrichtung verbunden. Dieses Prinzip lässt sich entsprechend auch auf eine Vielzahl von Mikrofluidvorrichtungen anwenden. Auf diese Weise kann die Anzahl der unterschiedlichen Flüssigkeiten weiter erhöht und die Flexibilität gesteigert werden.

Zwischen dem dritten fluidischen Reservoir und der Beobachtungskammer, sowie zwischen dem vierten fluidischen Anschluss und der Beobachtungskammer kann jeweils ein weiterer Ventilsitz angeordnet sein. Diese weiteren Ventilsitze können ebenfalls im Substrat ausgebildet sein.

Auf diese Weise können zwischen der Beobachtungskammer und dem dritten und vierten fluidischen Anschluss zwei weitere Ventile vorgesehen sein, sodass die Beobachtungskammer von diesen beiden Anschlüssen durch Schließen dieser Ventile abgeschirmt werden kann. Dies hat den Vorteil, dass kontrollierbar ist, ob ein Fluid von dem dritten bzw. vierten Flüssigkeitsreservoir in die Beobachtungskammer strömen soll oder nicht. Dies ermöglicht weitergehende Flexibilität und genauere Kontrolle des Flusses in der Mikrofluidvorrichtung.

Überdies können die vier Ventilsitze in Serie entlang des Kanalsystems angeordnet sein, wobei die Beobachtungskammer parallel zu den in Serie geschalteten Ventilsitzen angeordnet ist.

Mit dieser Anordnung ist es möglich, wie auch bereits zuvor beschrieben, ein Fluid in dem Kanal auszutauschen (vor allem eine Flüssigkeit mit Hilfe von Gas aus dem Kanalsystem zu spülen), ohne die Kultivierungsbedingungen in der Beobachtungskammer zu beeinflussen. Zu diesem Zweck werden die mit Hilfe der Ventilsitze gebildeten Ventile zwischen der Beobachtungskammer und dem Kanalsystem, also zwischen Zulauf und Kanalsystem, sowie zwischen Ablauf und Kanalsystem, so geschaltet, dass das Fluid nicht durch die Beobachtungskammer strömt. Alle anderen Ventile sind währenddessen im geöffneten Zustand. Nun kann ein Fluidaustausch vorgenommen werden, indem ein Gas durch das Kanalsystem geleitet wird, um die vorherige Flüssigkeit aus dem Kanalsystem zu verdrängen. Da die Beobachtungskammer durch die geschlossenen Ventilsitze von dem Kanalsystem abgetrennt ist, beeinflusst der Fluidaustausch nicht die Beobachtungskammer. In der Beobachtungskammer kultivierte Zellen erfahren damit keinen durch den Fluidaustausch bedingten Fluss (z.B. eine Trockenlegung durch die Verdrängung der Flüssigkeit) und die Kultivierungsbedingungen werden nicht beeinträchtigt.

Weiterhin stellt die vorliegende Erfindung ein Mikrofluidsystem bereit, das eine beschriebene Mikrofluidvorrichtung und eine Aktuatorvorrichtung umfasst, wobei die Mikrofluidvorrichtung und die Aktuatorvorrichtung zerstörungsfrei lösbar derart miteinander verbindbar sind, dass die Membran zwischen dem Substrat und der Aktuatorvorrichtung angeordnet ist, wobei die Aktuatorvorrichtung ausgebildet ist, eine Kraft auf die Membran auszuüben, wobei der erste Ventilsitz, die Membran und die Aktuatorvorrichtung ein erstes Ventil bilden, wobei der zweite Ventilsitz, die Membran und die Aktuatorvorrichtung ein zweites Ventil bilden, und wobei die Aktuatorvorrichtung mit der Mikrofluidvorrichtung derart zusammenwirkt, dass das erste Ventil und das zweite Ventil eine geschlossene Position und eine geöffnete Position aufweisen, wobei in der geschlossenen Position die Membran durch die Kraft in den jeweiligen Ventilsitz gedrückt wird, sodass das zugehörige Ventil in die geschlossene Position bringbar ist.

Das Mikrofluidsystem umfasst eine Mikrofluidvorrichtung und stellt damit auch deren Vorteile hinsichtlich der Zugabe von Fluiden bereit. Überdies wirken die Mikrofluidvorrichtung und die Aktuatorvorrichtung in einer bestimmungsgemäßen Verwendung zusammen, indem die genannten Ventile gebildet werden, die den Fluss in der Mikrofluidvorrichtung steuern.

Die Mikrofluidvorrichtung und die Aktuatorvorrichtung können über eine form- und/oder reibschlüssige Verbindung zusammengehalten werden, beispielsweise über eine Steckverbindung. Dazu können in dem Substrat Löcher ausgebildet sein. Eine Oberfläche der Aktuatorvorrichtung kann Stift aufweisen, die mit den Löchern im Substrat in Eingriff gebracht sind. Alternativ können die Mikrofluidvorrichtung und die Aktuatorvorrichtung mit Hilfe einer oder mehrerer Klemmen zusammengeklemmt werden. In beiden Fällen muss die Membran nicht zwingend eigens am Substrat befestigt sein. Stattdessen kann die Membran mit Hilfe einer Dichtlippe auf dem Substrat fixiert sein und sodann mit der Aktuatorvorrichtung verklemmt sein. Diese Art der Befestigung kommt gänzliche ohne Klebstoffe oder chemische Behandlung des Substrats und/oder der Membran aus. Bei Bedarf kann die Membran auch einfach ausgetauscht werden.

Die Aktuatorvorrichtung kann ebenso viele Verschließelemente umfassen wie die Mikrofluidvorrichtung Ventilsitze aufweist. Eines, mehrere oder jedes der Verschließelemente kann ein Kolben oder Stößel sein, und wobei jedes der Ventile durch einen der Ventilsitze, das zugehörige Verschließelement und die Membran gebildet wird. Weiterhin kann jedes Verschließelement unabhängig von den anderen Verschließelementen angetrieben werden.

Jeder Kolben bzw. Stößel kann ein- und ausgefahren werden. Im ausgefahrenen Zustand drückt der Kolben bzw. Stößel auf die elastische Membran und drückt diese in den entsprechenden Ventilsitz. Auf diese Weise wird das Ventil verschlossen. Wird der Kolben bzw. Stößel wieder eingefahren, kehrt die elastische Membran in ihre vorherige Position zurück und das Ventil ist im geöffneten Zustand. Jeder Kolben bzw. Stößel der Aktuatorvorrichtung wirkt mit einem Ventilsitz der Mikrofluidvorrichtung zusammen. Die Kombination aus einem Kolben bzw. Stößel und einer elastischen Membran zur Realisierung eines Ventils stellt dabei eine einfache, reversible und über viele Schaltzyklen zuverlässige Ausführung eines Ventils dar.

Eine der Membran zugewandte Seite jedes Kolbens oder Stößels kann abgerundet sein. Einer, mehrere oder jeder der Ventilsitze kann insbesondere angefast sein.

Die abgerundete Oberfläche des Kolbens bzw. Stößels weist keine scharfen Kanten auf und reduziert damit das Risiko einer Beschädigung der Membran.

Der angefaste Ventilsitz wird durch einen Fasenwinkel charakterisiert, der die Neigung des Ventilsitzes gegenüber der zweiten Seite des Substrats angibt. 0° und 90° bezeichnen den Grenzfall eines flachen Ventilsitzes. Der Fasenwinkel kann in einem Bereich zwischen 20° und 70°, insbesondere zwischen 30° und 60° liegen. In diesem Intervall hat sich herausgestellt, dass die Ventile im geschlossenen Zustand gut abdichten und diese Eigenschaft auch nach einer Vielzahl von Schaltzyklen behalten. Die Fase führt weiterhin dazu, dass der abgerundete Kolben bzw. Stößel stets in eine gewünschte Mittelposition auf dem Ventilsitz geführt wird. Der Stößel bzw. Kolben ist damit selbstpositionierend.

Alternativ können die der Membran zugewandte Seite des Kolbens bzw. Stößel und/oder der Ventilsitz flach ausgebildet sein. Auch in diesem Fall kann eine Abdichtung des Ventils im geschlossenen Zustand erreicht werden.

Die Aktuatorvorrichtung kann eine Antriebseinheit umfassen, die ausgebildet ist, die Verschlie-βelemente anzutreiben. Der Antrieb kann elektromagnetisch, hydraulisch, elektromechanisch oder pneumatisch sein.

Die Kolben bzw. Stößel der Aktuatorvorrichtung lassen sich durch diese Formen des Antriebs über eine große Anzahl an Schaltzyklen zuverlässig antreiben. Der Antrieb kann rein translatorisch sein. Die Verschließelemente können jeweils über eine Führung verfügen, wodurch die einzelnen Verschließelemente in Position gehalten werden und entlang einer vorgegebenen Linie geführt werden. Es ist auch ein Antrieb mittels eines Elektromotors denkbar. In diesem Fall erfolgt der Antrieb durch eine Superposition aus Translation und Rotation, wobei die Verschließelemente in Form einer Schraube ausgebildet sind und in einer Führung mit einem Gewinde, oder anders gearteten Getrieben, geführt werden.

Das beschriebene Mikrofluidsystem, wobei dieses Mikrofluidsystem Flüssigkeitsreservoire umfasst, kann weiterhin eine Druckluftvorrichtung umfassen, die ausgebildet ist, eines, Flüssigkeit aus einem, mehreren oder jedem der Flüssigkeitsreservoire mittels Überdruck oder Unterdruck zu fördern. Dazu können die Flüssigkeitsreservoire insbesondere unabhängig voneinander mit Druckluft (Überdruck und Unterdruck einschließend) beaufschlagt werden. Mit Hilfe der Druckluft kann die Flüssigkeit, durch das Mikrofluidsystem gepumpt werden. Ein Vorteil des Förderns mittels Druckluft besteht darin, dass das Fluid (die Flüssigkeit) nicht mit einer Pumpe und ihren Komponenten in Kontakt kommt, was das Risiko einer Kontamination der Flüssigkeit reduziert. Außerdem wird das Totvolumen an Flüssigkeit reduziert, weil die Flüssigkeit direkt vom Flüssigkeitsreservoir in das Kanalsystem gefördert werden kann statt beispielsweise eine Pumpe durchlaufen zu müssen.

Zwischen der Druckluft und der Flüssigkeit, insbesondere an der Phasengrenze, können Sterilfilter angeordnet sein. Diese Sterilfilter verhindern eine Kontamination der Flüssigkeit über die Druckluft.

Das Mikrofluidsystem kann weiterhin eine Steuereinrichtung zur Steuerung der Aktuatorvorrichtung umfassen. Die Steuervorrichtung kann ausgebildet sein, Antriebseinheit der Verschließelemente anzusteuern und damit das Öffnen und Schließen der Ventile zu steuern. Die Ansteuerung kann für jedes Verschließelement individuell sein, also unabhängig sein. Die Steuervorrichtung kann die Ansteuerung der Verschließelemente nach einer vorgegebenen Sequenz durchzuführen. Insbesondere kann die Steuereinrichtung zu diesem Zweck programmierbar sein. Alternativ oder zusätzlich kann die Steuereinrichtung ausgebildet sein, die Druckbeaufschlagung der Flüssigkeitsreservoire mittels der Druckluftvorrichtung vorzunehmen. Dadurch wird eine weitere Flexibilität der Ansteuerung des Mikrofluidsystems erreicht. Weiterhin kann die Steuerung durch die Steuerungseinheit automatisiert sein. Dadurch erhält man nach vorangegangener Programmierung ein weitgehend oder vollkommen autonom funktionierendes Mikrofluidsystem. Insbesondere erfolgt die Versorgung der Beobachtungskammer mit Flüssigkeit, Nährstoffen etc. automatisiert. Dies erhöht nicht nur die Einfachheit der Bedienung, sondern auch die Reproduzierbarkeit von experimentellen Ergebnissen gegenüber einem manuellen Antrieb.

Die Aktuatorvorrichtung kann eine kleinere Fläche als die Mikrofluidvorrichtung aufweisen, sodass die Mikrofluidvorrichtung über die Aktuatorvorrichtung hinausragt, wenn die Mikrofluidvorrichtung und die Aktuatorvorrichtung miteinander verbunden sind, wobei die Beobachtungskammer im Substrat der Mikrofluidvorrichtung ausgebildet ist, und wobei sich die Beobachtungskammer in dem Teil der Mikrofluidvorrichtung befindet, der über die Aktuatorvorrichtung hinausragt. Mit anderen Worten, wenn die Mikrofluidvorrichtung mit einer im Substrat ausgebildeten Beobachtungskammer und die Aktuatorvorrichtung miteinander verbunden sind, überlappt die Beobachtungskammer nicht mit der Aktuatorvorrichtung.

In der Regel befinden sich die Zellen, die untersucht werden sollen, in der Beobachtungskammer. Entsprechend soll es möglich sein, Mikroskopie an den Zellen in der Beobachtungskammer durchzuführen. Da die Aktuatorvorrichtung nicht mit der Beobachtungskammer überlappt, kann stattdessen ein Objektiv in unmittelbarer Nähe zur Beobachtungskammer angeordnet sein, um Mikroskopie mit hoher Auflösung zu erlauben.

Die Mikrofluidvorrichtung kann ein Wegwerfartikel für den Einmalgebrauch sein. Die Aktuatorvorrichtung kann wiederverwendbar sein.

Das Fluid ist lediglich in Kontakt mit der Mikrofluidvorrichtung, aber nicht mit der Aktuatorvorrichtung. Damit sind Teile, die mit dem Fluid in Berührung kommen, für den Einmalgebrauch ausgelegt. Nach der Benutzung kann die Mikrofluidvorrichtung einfach entsorgt werden und muss nicht gereinigt werden. Außerdem kann die Sterilität der Komponenten, die mit dem Fluid in Berührung kommen, leicht gewahrt bleiben, indem für eine neue Anwendung auch eine neue Mikrofluidvorrichtung verwendet wird. Falls die Mikrofluidvorrichtung mittels Spritzguss hergestellt wird, kann sie günstig und in großer Stückzahl produziert werden. Hingegen kann die Aktuatorvorrichtung mit den Verschließelementen etc. mehrfach verwendet werden. Sie kommt nicht mit dem Fluid in Kontakt und muss daher auch nicht nach jeder Benutzung gereinigt werden und sorgt ebenso wenig für eine potentielle Kontamination des Fluids.

Die Aktuatorvorrichtung kann mit einer Vielzahl verschiedener Mikrofluidvorrichtungen verwendbar sein, also eine universelle Aktuatorvorrichtung sein. Dazu umfasst die Aktuatorvorrichtung eine Mehrzahl an Verschließelementen an vordefinierten Positionen. Die Mikrofluidvorrichtung ist auf diese Aktuatorvorrichtung insofern abgestimmt, als dass die Ventilsitze ebenfalls an vordefinierten Positionen im Substrat vorgesehen sind und mit den Verschließelementen zusammenwirken. Die universelle Aktuatorvorrichtung umfasst mehr oder gleich viele Verschließelemente wie die Mikrofluidvorrichtung Ventilsitze umfasst. Für den Fall, dass weniger Ventilsitze als Verschlie-βelemente vorliegen, werden in der Aktuatorvorrichtung jene Verschließelemente nicht angesteuert, die mit keinem Ventilsitz zusammenwirken. Mit dieser Anordnung kann also eine Aktuatorvorrichtung mit einer Vielzahl verschiedener Mikrofluidvorrichtungen verwendet werden. Dies ist insbesondere deshalb sinnvoll, weil die Aktuatorvorrichtung ein wiederverwendbarer Artikel ist und für eine Mehrzahl an Verwendungszyklen vorgesehen ist und/oder mit einer Vielzahl von Mikrofluidvorrichtungen zusammenwirken soll.

Weitere Merkmale und Vorteile der Erfindung werden nachfolgend anhand der beispielhaften Figuren erläutert. Dabei zeigen:
- Figur 1A: eine Schrägansicht einer Mikrofluidvorrichtung;
- Figur 1B: eine weitere Schrägansicht der Mikrofluidvorrichtung gemäß Fig. 1A;
- Figur 1C: eine Explosionsansicht der Mikrofluidvorrichtung gemäß Fig. 1A;
- Figur 2A: eine Schrägansicht eines Mikrofluidsystems;
- Figur 2B: ein Blockschaltbild eines Mikrofluidsystems gemäß Figur 2A;
- Figur 3: eine Detailansicht eines Ventils in einem Mikrofluidsystem;
- Figur 4A: ein schematisches Schaltbild eines Mikrofluidsystems;
- Figur 4B: einen vereinfachten Schaltplan der Ventilanordnung in einem Mikrofluidsystem gemäß Figur 4A
- Figur 4C: eine schematische Darstellung der Aktuatorvorrichtung des Mikrofluidsystems der Figur 4A;
- Figur 5A: ein schematisches Schaltbild eines Mikrofluidsystems;
- Figur 5B: einen vereinfachten Schaltplan der Ventilanordnung in einem Mikrofluidsystem gemäß Figur 5A;
- Figur 5C: eine schematische Darstellung der Aktuatorvorrichtung des Mikrofluidsystems der Figur 5A;
- Figur 6: eine Schrägansicht eines Mikrofluidsystems; und
- Figur 7: eine Schrägansicht eines weiteren Mikrofluidsystems.

Im Folgenden und in den Figuren werden in den verschiedenen Ausführungsbeispielen, sofern nicht anders angegeben, die gleichen Bezugszeichen für gleiche oder entsprechende Elemente verwendet.

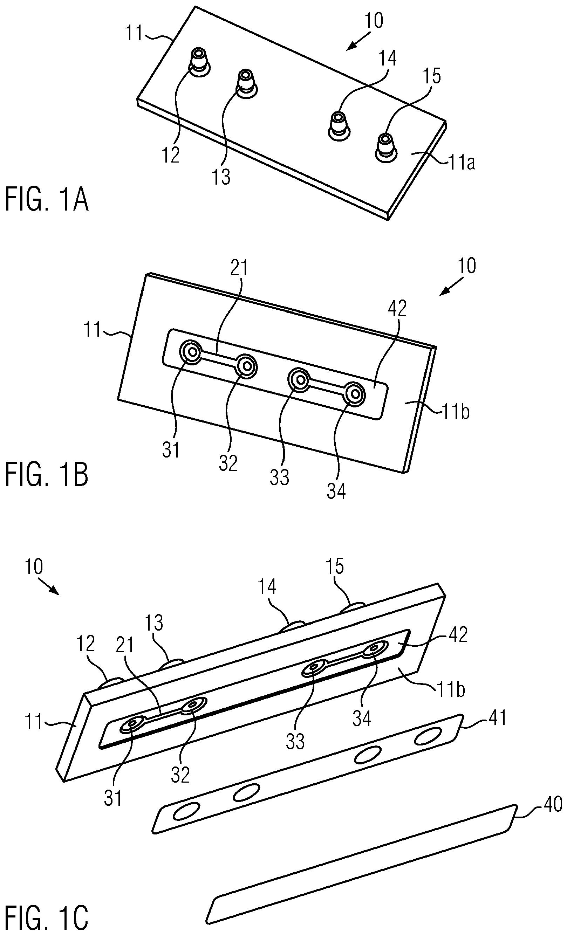

Figuren 1A bis 1C zeigen verschiedene Ansichten einer erfindungsgemäßen Mikrofluidvorrichtung 10. Diese umfasst zunächst ein Substrat 11, einen ersten fluidischen Anschluss 12, einen zweiten fluidischen Anschluss 13, einen dritten fluidischen Anschluss 14, einen vierten fluidischen Anschluss 15 und einen Graben 21.

Die Mikrofluidvorrichtung 10 ist für die Verwendung mit Flüssigkeiten und Gasen geeignet.

Figur 1A zeigt eine schematische Schrägansicht der Mikrofluidvorrichtung 10, wobei eine erste Seite 11a des Substrats 11 nach oben weist. An dieser ersten Seite 11a sind auch die vier fluidischen Anschlüsse 12, 13, 14, 15 angeordnet. Die Anschlüsse 12, 13, 14, 15 können an dem Substrat 11 befestigt sein, sie können aber auch integraler Bestandteil des Substrats 11 sein. In letzterem Fall sind die Anschlüsse 12, 13, 14, 15 und das Substrat 11 aus dem gleichen Material und insbesondere aus einem Stück gefertigt. Dies bringt den Vorteil höherer Stabilität und der Möglichkeit einer vereinfachten Herstellung, vor allem, weil weniger Handhabungsschritte nötig sind, um die Mikrofluidvorrichtung 10 zusammenzusetzen. So ist es beispielsweise möglich, das Substrat 11 einschließlich der Anschlüsse 12, 13, 14, 15 mittels Spritzguss in einem Herstellungsschritt zu produzieren.

Das Substrat 11 kann im sichtbaren Wellenlängenbereich transparent sein. In einigen Anwendungen genügt es hingegen auch, wenn das Substrat 11 lediglich transluzent oder sogar opak ist, insbesondere dann, wenn keine optischen Untersuchungen an dem Substrat 11 vorgenommen werden müssen.

Figur 1B zeigt eine schematische Schrägansicht der Mikrofluidvorrichtung 10, wobei eine zweite Seite 11b des Substrats 11 nach oben weist, wobei die zweite Seite 11b der ersten Seite 11a gegenüberliegt. Zur Vereinfachung ist in dieser Darstellung keine Membran gezeigt, sondern erst in Figur 1C. In dieser zweiten Seite 11b befindet sich eine Vertiefung in der Oberfläche des Substrats 11. Diese Vertiefung dient der Aufnahme einer Membran. Ebenfalls befindet sich in der zweiten Seite 11b ein Graben 21. Durch Abdecken mit einer Folie (wird nachfolgend beschrieben) wird dieser Graben 21 zum Kanalsystem. Wo sich an der ersten Seite 11a ein Anschluss befindet, liegt gegenüber auf der zweiten Seite 11b ein Ventilsitz. Eine Flüssigkeit, die an einem der Anschlüsse eingegeben wird, fließt durch das Substrat 11 hindurch zur zweiten Seite und tritt durch den Ventilsitz. Den vier Anschlüssen 12, 13, 14 und 15 liegen auf der zweiten Seite 11b also die Ventilsitze 31, 32, 33, 34 gegenüber.

Der Graben 21, der an der zweiten Seite 11b des Substrats 11 ausgebildet ist, verbindet jeweils ein Paar von Ventilsitzen miteinander, sodass das Fluid von einem Ventilsitz durch das Kanalsystem zu einem anderen Ventilsitz strömen kann.

Die Struktur des Kanalsystems bzw. des Grabens 21 kann für jede Mikrofluidvorrichtung individuell ausgebildet sein, und weitere Beispiele sind mit Bezug auf die nachfolgenden Ausführungsformen erläutert.

Figur 1C ist eine Explosionsansicht der Mikrofluidvorrichtung 10. Die Mikrofluidvorrichtung 10 umfasst das zuvor erläuterte Substrat 11 (mit den Anschlüssen und Ventilsitzen) und eine elastische Membran 40. Die Membran 40 wird mit Hilfe einer doppelseitigen Klebefolie 41 an dem Substrat 11 angebracht. In der zweiten Seite 11b des Substrats 11 ist eine Vertiefung 42 ausgebildet, deren Form der Form der Membran 40 und der Klebefolie 41 entspricht. Die Klebefolie 41 wird in der Vertiefung 42 platziert. Dabei spart die Klebefolie 41 die Ventilsitze 31, 32, 33, 34 aus, weshalb in der Klebefolie 41 die kreisrunden Löcher vorgesehen sind (die Löcher werden an die Form der Ventilsitze angepasst. Da die Ventilsitze im gezeigten Fall rund sind, gilt das auch für die Löcher in der Klebefolie 41). Die Membran 40 wird auf der in der Vertiefung 42 fixierten Klebefolie 41 platziert und darauf festgeklebt. Insofern wird der Graben 21 durch die Klebefolie 41 abgedeckt und das Kanalsystem 20 gebildet.

Die Einzelteile dieser Mikrofluidvorrichtung 10, also das Substrat 11, die Klebefolie 41 und die Membran 40 sind einfach herstellbar und der Zusammenbau der Mikrofluidvorrichtung 10 ist ebenfalls unkompliziert.

Alternativ kann die Membran 40 auch ohne Klebefolie 41 in der Aussparung platziert werden. In diesem Fall wird die Membran in die Aussparung 42 geklemmt.

Die Klebefolie 41 kann neben den Ventilsitzen auch den Graben 21 aussparen. In diesem Fall wird das Kanalsystem durch die Abdeckung des Grabens 21 mit der Membran 40 gebildet. Dadurch können Zellen, die durch das Kanalsystem 20 geleitet werden, nicht an der Klebefolie 41 anhaften. Ebenso werden Substanzen von der Oberfläche der Klebefolie (z.B. der klebrige Film) nicht in den Fluidstrom im Kanalsystem abgegeben, was eine mögliche unerwünschte Kontamination vermeidet.

Die Vertiefung 42 hat insbesondere die gleiche Dicke/Tiefe wie die Klebefolie 41 und die Membran 40 in Summe. Dadurch ist die Unterseite der Mikrofluidvorrichtung plan, wenn die Mikrofluidvorrichtung 10 zusammengesetzt wurde. Dies hat den Vorteil, dass für mikroskopische Untersuchungen eine plane Oberfläche zur Verfügung steht, was die optischen Untersuchungen positiv beeinflusst. Falls keine Klebefolie verwendet wird, entspricht die Tiefe der Vertiefung 42 der Dicke der Membran 40.

Die Membran 40 kann eine Silikonmembran sein und insbesondere Elastosil umfassen oder daraus bestehen. Ebenso kann die Membran ein thermoplastisches Elastomer wie Flexdym umfassen oder daraus bestehen. Alternativ kann die Membran auch ein COC umfassen oder daraus bestehen. Die Dicke der Membran 40 liegt im Bereich zwischen 5 µm und 5 mm. Damit besitzt die Membran eine hohe Langlebigkeit über eine Vielzahl von Schaltzyklen hinweg und man benötigt gleichzeitig nur eine geringe Kraft, um die Membran 40 in den zugehörigen Ventilsitz zu drücken.

Diese Mikrofluidvorrichtung 10 erlaubt es, über ihre fluidischen Anschlüssen gezielt Fluide in das Kanalsystem einzugeben. Die Zugabe wird dabei durch den zugehörigen Ventilsitz gesteuert, wobei der Ventilsitz bei Bedarf durch die Membran geschlossen und die Zugabe von Fluid blockiert werden kann. Eine Flüssigkeit, die sich bereits in dem Kanalsystem befindet, wird durch die Zugabe weiterer Flüssigkeiten vermischt, aber ein bestehender Fluss wird nicht behindert. Das ist insbesondere von Vorteil, wenn dieser Fluss zur Kultivierung mikrobiologischer Proben benutzt wird, da eine ungewünschte Beeinträchtigung der Kultivierungsbedingungen mit Hilfe dieser Mikrofluidvorrichtung 10 vermieden werden kann. Darüber hinaus ist die vorliegende Mikrofluidvorrichtung überdies einfach und kompakt aufgebaut. Mit anderen Worten, die Mikrofluidvorrichtung ermöglicht eine effiziente und präzise Kontrolle des Fluidflusses in einem Kanalsystem bei gleichzeitig kompaktem und einfachem Aufbau.

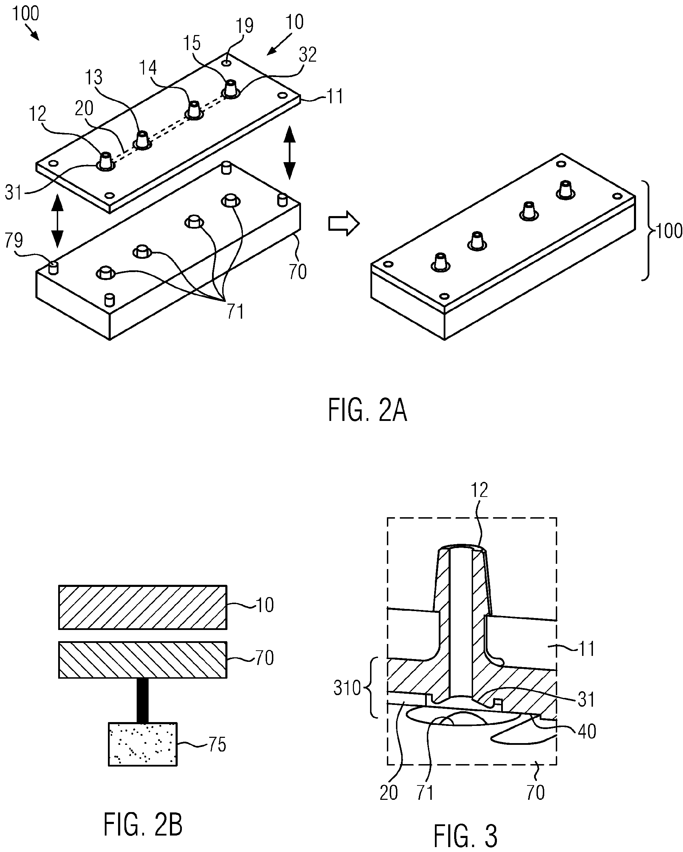

Figur 2A zeigt die Schrägansicht eines Mikrofluidsystems 100. Dieses umfasst eine Mikrofluidvorrichtung 10 und eine Aktuatorvorrichtung 70. Die Mikrofluidvorrichtung 10 kann wie in Figur1A-C illustriert ausgebildet sein.

Die Aktuatorvorrichtung 70 umfasst eine Mehrzahl an Verschließelementen 71, die im vorliegenden Fall als Kolben oder Stößel ausgebildet sind. Das Verschließelement 71 kann elektromechanisch, elektromagnetisch, hydraulisch oder pneumatisch, beispielsweise durch ein Druckluftventil, angetrieben werden. Optional ist der Aktuator bistabil und verfügt über einen Verriegelungsmechanismus. Die Verschließelemente 71 können ausgefahren und eingefahren werden, wobei die Verschließelemente 71 in der Figur im ausgefahrenen Zustand gezeigt sind. Zum Antrieb der Antriebselemente 71 dient eine entsprechende Antriebseinheit 75 (siehe Figur 2B), die beispielsweise ein Servomotor, ein Elektromotor, ein Hubmagnet oder ein pneumatischer oder hydraulischer Zylinder sein kann. Ein bistabiler Aktuator nimmt in einem stromlosen Zustand verschiedene stabile Schaltzustände ein. Die stabilen Zustände können durch eine mechanische Verriegelung, magnetische Remanenz oder einen Permanentmagneten realisiert werden. Im Fall des Hubmagneten werden die stabilen Zustände über einen Permanentmagneten und eine Rückstellfeder erzeugt.

Im Blockschaltbild der Figur 2B ist dieser Aufbau eines Mikrofluidsystems 100 mit einer Mikrofluidvorrichtung 10, einer Aktuatorvorrichtung 70 und einer Antriebseinheit 75 gezeigt.

Die Mikrofluidvorrichtung 10 und die Aktuatorvorrichtung 70 sind gemäß Figur 2A miteinander verbindbar, wobei die Verbindung zerstörungsfrei lösbar ist. Beispielsweise können die Mikrofluidvorrichtung 10 und die Aktuatorvorrichtung 70 zusammengesteckt werden und durch einen Formschluss sicher, aber auch lösbar, verbunden sein. Alternativ können die beiden Vorrichtungen durch zusätzliche Klammern (nicht dargestellt) geklemmt werden. In der Figur weist die Aktuatorvorrichtung 70 eine Mehrzahl von Stiften 79 auf. Gleichzeitig weist das Substrat 11 eine Mehrzahl von Löchern 19 auf. Beim Zusammensetzten des Mikrofluidsystems 100 werden die Stifte 79 in die Löcher 19 gesteckt und so die genannte formschlüssige, dennoch zerstörungsfrei lösbare, Verbindung (Steckverbindung) zwischen der Mikrofluidvorrichtung 10 und der Aktuatorvorrichtung 70 hergestellt.

Wenn die Mikrofluidvorrichtung 10 und die Aktuatorvorrichtung 70 verbunden sind, ist die Membran 40 zwischen dem Substrat 11 und der Aktuatorvorrichtung 70 angeordnet. Weiterhin sind die Mikrofluidvorrichtung 10 und die Aktuatorvorrichtung 70 derart aufeinander angepasst, dass jeder der Ventilsitze 31, 32, 33, 34 mit einem der Verschließelemente überlappt. Ein Verschließelement 71, die Membran 40 und ein Ventilsitz 31, 32, 33, 34 bilden in dieser Konfiguration ein Ventil, wobei jedes der Antriebselemente 71 einem Ventilsitz 31, 32, 33, 34 zugeordnet ist. Die Funktion eines so gebildeten Ventils wird mit Bezug auf Figur 3 genauer erläutert.

Die Mikrofluidvorrichtung 10 und die Aktuatorvorrichtung 70 können die gleichen Abmessungen ihres Querschnitts aufweisen. Beispielsweise kann die Mikrofluidvorrichtung die Abmessungen 35 cm mal 35 cm, insbesondere 15 cm mal 20 cm, weiterhin insbesondere 5 cm mal 5 cm aufweisen. Ebenso können die Abmessungen einem ANSI/SLAS-Standard für Multiwell-Platten entsprechen. Die Aktuatorvorrichtung 70 kann die gleiche Länge und Breite wie die Mikrofluidvorrichtung 10 aufweisen.

In Figur 3 ist eine Detailansicht eines Ventils 310, wie es sich in einem Mikrofluidsystem 100 ergibt. Das Mikrofluidsystem 100 umfasst eine Mikrofluidvorrichtung 10 und eine Aktuatorvorrichtung 70, die miteinander verbindbar sind. In gezeigtem Fall, der eine bestimmungsgemäße Verwendung des Mikrofluidsystems 100 beschreibt, sind die beiden Komponenten verbunden.

Die Mikrofluidvorrichtung 10 umfasst ein Substrat 11, einen Anschluss 12 an der ersten Seite 11a des Substrats 11, und einen Ventilsitz 31 auf der zweiten Seite 11b des Substrats 11, wobei die zweite Seite 11b der ersten Seite 11a gegenüberliegt. Durch das Substrat 11 hindurch führt eine Durchgangsöffnung vom Anschluss 12 zum Ventilsitz 31. Der Ventilsitz 31 selbst ist angefast, also trichterförmig.

Die Aktuatorvorrichtung 70 umfasst ein Verschließelement 71 in Form eines Stößels. Der Stößel weist eine abgerundete Oberfläche auf, die der Membran 40 zugewandt ist. In der Figur ist das Verschließelement 71 in einem eingezogenen Zustand gezeigt, bei dem der Kolben in der Aktuatorvorrichtung 70 versenkt ist. Das Verschließelement 71 kann in einen ausgefahrenen Zustand gebracht werden, beispielsweise, durch einen elektromechanischen, elektromagnetischen oder hydraulischen Antrieb. Der Stößel wird durch den angefasten Ventilsitz stets automatisch in eine zentrale Position geführt, um den Ventilsitz optimal abzudichten. Der Ventilsitz wirkt für den Stö-ßel also selbstpositionierend.

Der Ventilsitz 31 kann auch abgerundet sein und mit einer ebenfalls abgerundeten Oberfläche des Verschließelements 71 zusammenwirken.

Die Erfindung ist allerdings nicht auf diese Form Verschließelements 71 und Ventilsitzes 31 beschränkt. Alternativ kann der Ventilsitz flach ausgebildet sein. Dazu passend kann auch das Verschließelement 71 in Form eines Stößels mit einer flachen Oberfläche ausgebildet sein. Denkbar sind auch abgerundete Kanten an der flachen Stößeloberfläche, um eine mögliche Verletzung der Membran durch scharfe Kanten zu vermeiden.

Aus dieser Darstellung ergibt sich auch die Funktionsweise des Ventils 310 und des Mikrofluidsystems 100. Im ausgefahrenen Zustand übt das Verschließelement 71 durch Druck eine Kraft auf die Membran 40 aus und drückt diese in den Ventilsitz 31, wodurch das Ventil im geschlossenen Zustand ist. Der angefaste Ventilsitz 31 im Zusammenspiel mit der abgerundeten Oberfläche des Verschließelements trägt zu einer besseren Abdichtung im geschlossenen Zustand des Ventils 310 bei. Im geschlossenen Zustand der Ventils 310 kann folglich kein Fluid vom Anschluss 12 in das Kanalsystem 20 gelangen oder umgekehrt. Sobald das Verschließelement 71 in den eingefahrenen Zustand gebracht wird, kehrt die Membran 40 aufgrund ihrer Elastizität in ihre vorherige Position zurück und das Ventil 310 ist in den offenen Zustand gebracht. Nun kann ein Fluid vom Anschluss 12 in das Kanalsystem 20 fließen oder vom Kanalsystem 20 zum Anschluss 12.

Das Kanalsystem 20 ist weiterhin so gestaltet, dass ein Kanal unterhalb des Ventilsitzes 31 liegt, sich dort aber aufweitet, sodass er durch das Verschließen des Ventils 310 nicht ebenfalls verschlossen wird. Das bedeutet, dass ein Fluid um den Ventilsitz 31 fließen kann und dabei im Kanalsystem 20 verbleibt, wenn das Ventil 310 geschlossen ist. Wenn das Ventil 310 geöffnet ist, kann das Fluid von dem zugehörigen fluidischen Anschluss durch den Ventilsitz 31 in das Kanalsystem 20 eintreten. Das heißt, dass ein geschlossenes Ventil 310 nicht zum Stopp des Flusses führen muss, sondern das Fluid kann weiter im Kanalsystem 20 verbleiben, ohne durch den Ventilsitz 31 zu strömen.

Mit einem oder mehreren der fluidischen Anschlüsse können Flüssigkeitsreservoire verbunden sein, sodass bei geöffneten Ventilen die Flüssigkeit aus dem jeweiligen Flüssigkeitsreservoir in das Kanalsystem fließt. Durch gezieltes Öffnen und Schließen der Ventile wird gesteuert, welche Flüssigkeiten in das Kanalsystem fließen. Über eine Kontrolle der Öffnungsdauer kann auch die Menge der jeweiligen Flüssigkeit, die in das Kanalsystem eingeleitet wird, gesteuert werden. Es kann damit ein präzises Mischungsverhältnis von Flüssigkeiten in dem Kanalsystem hergestellt werden. Weiterhin kann das Kanalsystem mit einer Beobachtungskammer verbunden sein, in der sich eine mikrobiologische Probe befindet. Diese Probe wird mit dem Flüssigkeitsgemisch aus dem Kanalsystem versorgt. Handelt es sich bei den Flüssigkeiten beispielsweise um Nährlösungen, kann eine präzise Versorgung der Probe mit Nährstoffen erfolgen. Insofern kann das Mikrofluidsystem gemäß Figur 2 für die Bereitstellung einer präzise abgestimmten Nährlösung für mikrobiologische Proben sein.

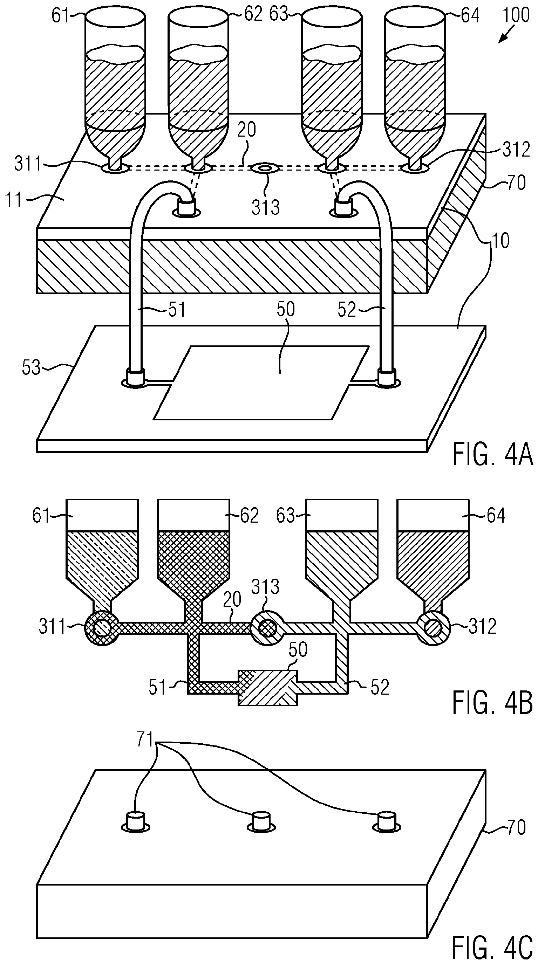

Eine andere Ausführungsform des Mikrofluidsystems 100 ist in Figur 4 gezeigt. Diese basiert im Wesentlichen auf der ersten Ausführungsform (Figur 2A), umfasst also eine Mikrofluidvorrichtung 10 und eine Aktuatorvorrichtung 70. Die Mikrofluidvorrichtung 10 umfasst ein Substrat 11, wobei in dem Substrat 11 ein Kanalsystem 20 und drei Ventilsitze ausgebildet sind, sowie eine Membran. Die Aktuatorvorrichtung 70 umfasst drei Verschließelemente 71, die mit den drei Ventilsitzen zusammenwirken, wenn die Mikrofluidvorrichtung 10 und die Aktuatorvorrichtung 70 miteinander verbunden sind.

Das Mikrofluidsystem 100 verfügt über vier Flüssigkeitsreservoire 61, ..., 64, die direkt auf die Anschlüsse der Mikrofluidvorrichtung 10 gesteckt sind. Zwischen dem ersten Flüssigkeitsreservoir 61 und dem Kanalsystem 20, sowie zwischen dem vierten Flüssigkeitsreservoir 64 und dem Kanalsystem befinden sich die Ventile 311 und 312, sodass das Fluid von dem jeweiligen Reservoir nur dann in das Kanalsystem 20 strömen kann, wenn das jeweilige Ventil geöffnet ist. Fluid von dem zweiten Flüssigkeitsreservoir 62 und dem dritten Flüssigkeitsreservoir 63 kann hingegen direkt in das Kanalsystem 20 eintreten, da hier kein Ventil angeordnet ist. Das Ventil 311 wird durch den Ventilsitz 31, eines der Antriebselemente 71 und die Membran gebildet. Das Ventil 312 wird durch den Ventilsitz 32, eines der Antriebselemente 71 und die Membran gebildet.

Durch das direkte Aufstecken der Flüssigkeitsreservoire 61-64 auf die Anschlüsse entfallen etwaige Schlauchverbindungen, sodass die Komplexität des gesamten Aufbaus reduziert werden kann. Damit einher geht eine vereinfachte Sterilhaltung der Vorrichtung, da weniger potentiell kontaminierbare Komponenten involviert sind. Ein weiterer Vorteil besteht darin, dass das Totvolumen reduziert wird, weil keine Schläuche mit der Flüssigkeit befüllt werden müssen. Es reicht also eine geringe Flüssigkeitsmenge aus, die direkt vom jeweiligen Flüssigkeitsreservoir 61-64 in das Kanalsystem 20 gefördert wird. Ein Wegfallen von Schlauchverbindungen hat darüber hinaus den Vorteil, dass etwaige Temperaturgradienten entlang des Transportwegs des Fluids nicht auftreten. Bei Flüssigkeiten reduziert dies die Möglichkeit von Blasenbildung. Luftblasen könnten negative Auswirkungen auf die Kultivierung der Zellen haben oder Untersuchungsergebnisse verfälschen.

Eine Beobachtungskammer 50 ist über Verbindungsschläuche mit dem Kanalsystem 20 verbunden, wobei der Schlauch 51 als Zufluss und der Schlauch 52 als Abfluss der Beobachtungskammer 50 dient (auch die umgekehrte Zuordnung ist möglich, wobei die Zuordnung grundsätzlich von der Flussrichtung abhängt). Die Beobachtungskammer 50 ist in einem separaten Substrat 53 ausgebildet und wird als Teil der Mikrofluidvorrichtung 10 angesehen.

Die Flüssigkeitsreservoire 62, 63 können beispielsweise eine Nährlösung enthalten, die durch die Beobachtungskammer 50 gefördert wird. In der Beobachtungskammer 50 können sich Zellen oder Zellaggregate befinden, die unter kontrollierten Umgebungsbedingungen kultiviert werden sollen. Durch Zuführung der Nährlösung werden die Zellen in der Beobachtungskammer 50 mit Nährstoffen versorgt. Wird das Ventil 311 geöffnet, kann eine weitere Flüssigkeit aus dem ersten Flüssigkeitsreservoir 61 in das Kanalsystem 20 eingeleitet werden, wo es sich mit der Nährlösung vermischt. Die Dosierung der weiteren Flüssigkeit kann durch die Dauer der Öffnung und/oder eine Druckbeaufschlagung des ersten Flüssigkeitsreservoirs 61 eingestellt flexibel und kontrolliert eingestellt werden. Gleiches gilt für eine weitere Flüssigkeit in dem vierten Flüssigkeitsreservoir 64. Auf diese Weise können einfach und kontrolliert weitere Flüssigkeiten in das Kanalsystem 20 eingeleitet werden, wo sie sich mit der Nährlösung vermischen und den Zellen in der Beobachtungskammer 50 zugeführt werden.

In dem Substrat 11 befindet sich ein weiterer Ventilsitz 33 in fluidischer Verbindung mit dem Kanalsystem 20, der parallel zu den Anschlüssen für die Beobachtungskammer 50 angeordnet ist. Dieser Ventilsitz 33 ist also in einem Bypass angeordnet, der es erlaubt, ein Fluid durch das Kanalsystem 20 an der Beobachtungskammer 50 vorbei zu fördern. Dieser Bypass ist Teil des Kanalsystems und kann auch als Bypass-Kanal bezeichnet werden. Zusammen mit der Aktuatorvorrichtung 70 und der Beobachtungskammer 50 wird so ein Ventil 313 gebildet, das parallel zur Beobachtungskammer 50 angeordnet ist. Dieses Ventil 313 kann als Bypass-Ventil bezeichnet werden. Über das Bypass-Ventil 313 kann eingestellt werden, ob das Fluid ausschließlich durch die Beobachtungskammer zwischen dem dritten Anschluss 14 und dem vierten Anschluss 15 fließen soll, oder ob ein Anteil des Fluids über den Bypass strömen soll. Beispielsweise kann durch Öffnen des Bypass-Ventils 313 der Fluss in der Beobachtungskammer 50 reduziert werden.

Ein schematisches Schaltbild dieses Mikrofluidsystems ist in Figur 4B gezeigt. Die beiden Flüssigkeitsreservoire 61, 64 sind durch das jeweils zugehörige Ventil 311, 312 vom Kanalsystem abgetrennt. Die anderen beiden Flüssigkeitsreservoire 62, 63 sind hingegen ohne Ventile direkt mit dem Kanalsystem verbunden. Dadurch kann auch die Flüssigkeit aus den Flüssigkeitsreservoiren 62, 63 direkt der Beobachtungskammer 50 zugeführt werden. Flüssigkeit aus den Flüssigkeitsreservoiren 61, 64 wird dem Kanalsystem 20 durch Öffnen des jeweiligen Ventils zugeführt. Ebenso ist die Funktion des Bypass-Ventils 313 genauer dargestellt. Wenn das Bypass-Ventil 313 geschlossen ist, strömt das Fluid auf dem Weg zwischen den Flüssigkeitsreservoiren 62, 63 ausschließlich durch die Beobachtungskammer 50. Durch Öffnen des Bypass-Ventils 313 hingegen kann ein Teil des Fluids auch über den Bypass strömen und der Fluss durch die Beobachtungskammer 50 wird reduziert.

Zur weiteren Illustration der Ventilanordnung und ihrer Funktion ist in Figur 4C die Aktuatorvorrichtung 70 separat von der Mikrofluidvorrichtung 10 gezeigt. Die Aktuatorvorrichtung 70 umfasst drei Verschließelemente 71, die mit den drei Ventilsitzen der Mikrofluidvorrichtung 10 zusammenwirken. Eine Querschnittsfläche des Substrats 11 entspricht dabei insbesondere einer Querschnittsfläche der Aktuatorvorrichtung 70. Die Mikrofluidvorrichtung 10 und die Aktuatorvorrichtung 70 können, wie zu Figur 2 erläutert, mit einer Steckverbindung zusammengesteckt werden.

Diese Konfiguration kann vor allem dann Anwendung finden, wenn sich in dem Fluid, das durch das Kanalsystem 20 und die Beobachtungskammer 50 gefördert werden soll, bestimmte Zelltypen wie Blutzellen oder Immunzellen befinden. Diese Zellen sollen insbesondere nicht thermisch oder mechanisch gestresst werden, weil die Zellen sonst aktiviert werden können, was im Allgemeinen nicht gewünscht ist. Ein solcher (v.a. mechanischer) Stress tritt insbesondere an einem Ventil auf, weil an einer solchen Engstelle leicht turbulente Strömung auftritt und die Zellen durch Kontakt mit dem Substrat, der Membran oder untereinander stark beansprucht werden. Dieser Stress kann in diesem Ausführungsbeispiel vermieden werden. Üblicherweise werden die Zellen zwischen dem dritten Flüssigkeitsreservoir 63 und dem vierten Flüssigkeitsreservoir 64 durch die Beobachtungskammer gefördert. Dieser Abschnitt ist frei von Ventilen, sodass der beschriebene Stress nicht oder in reduziertem Maß auftritt.

Diese Ausführungsform des Mikrofluidsystems kann im Prinzip dadurch erweitert werden, dass an Stelle einer einzelnen Mikrofluidvorrichtung und Aktuatorvorrichtung eine Kaskade vorgesehen ist. Dabei wird in dem beschriebenen Mikrofluidsystem statt der Beobachtungskammer eine weitere Mikrofluidvorrichtung angeordnet und die beiden Schläuche 51, 52 werden mit der weiteren Mikrofluidvorrichtung verbunden, wo die Schläuche an die Stelle der Flüssigkeitsreservoire 61, 64 treten. Die weitere Aktuatorvorrichtung wird wie zuvor beschrieben mit der Mikrofluidvorrichtung verbunden und die Beobachtungskammer kann mit der zweiten Mikrofluidvorrichtung verbunden werden. Natürlich kann dieses Prinzip entsprechend auf mehr als zwei Mikrofluidvorrichtungen und Aktuatorvorrichtungen erweitert werden. Dies bringt den Vorteil mit sich, dass mehr (unterschiedliche) Flüssigkeiten in das System eingespeist werden können und eine flexiblere Ausgestaltung möglich wird. Beispielsweise ermöglichen zwei in Serie geschaltete Mikrofluidvorrichtungen die Zugabe von bis zu sechs Flüssigkeiten, während eine solche Mikrofluidvorrichtung maximal vier Flüssigkeiten erlaubt.

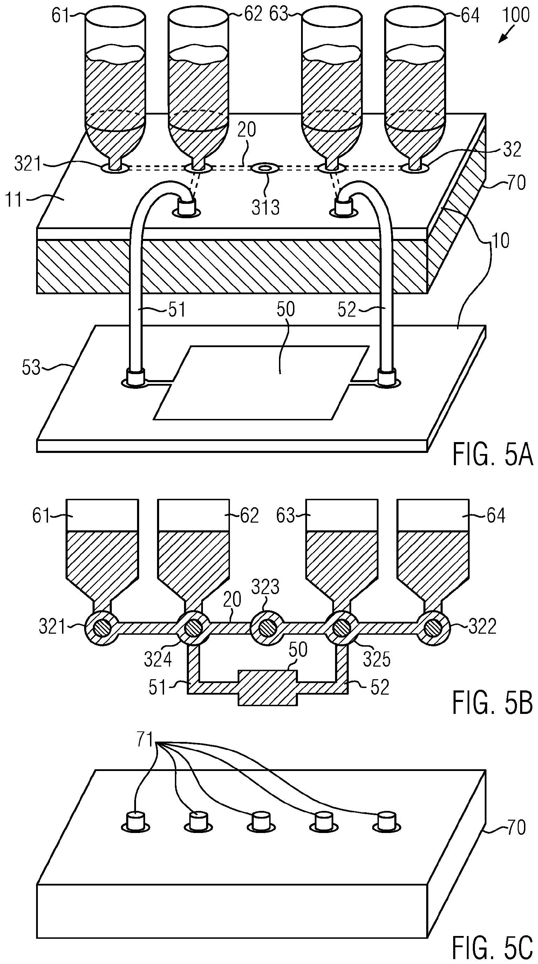

Figur 5A zeigt eine weitere Ausführungsform des Mikrofluidsystems 100. Sie unterscheidet sich von der zweiten Ausführungsform durch zwei zusätzliche Ventile 324 und 325, die zwischen dem dritten Anschluss 14 und der Beobachtungskammer 50, sowie zwischen dem vierten Anschluss 15 und der Beobachtungskammer 50 angeordnet sind. Im Übrigen umfasst auch diese Ausführungsform eine Mikrofluidvorrichtung 10 mit einem Substrat 11, Flüssigkeitsreservoiren 61, 62, 63, 64, einem Kanalsystem 20, einer Beobachtungskammer 50, sowie eine Aktuatorvorrichtung 70.

Zwischen dem zweiten Flüssigkeitsreservoir 62 und dem Kanalsystem 20, sowie zwischen dem dritten Flüssigkeitsreservoir 63 und dem Kanalsystem 20 ist jeweils ein Ventil 324 bzw. 325, die durch die Zusammenwirkung von jeweils einem Ventilsitz, der Membran und einem Verschlie-ßelement der Aktuatorvorrichtung gebildet werden. Durch Öffnen und Schließen dieser Ventile kann also ein Fluss zwischen dem zweiten Flüssigkeitsreservoir 62 und dem Kanalsystem 20, sowie zwischen dem dritten Flüssigkeitsreservoir 63 und dem Kanalsystem 20 kontrolliert werden (was in der Ausführungsform gemäß Figur 4 nicht möglich ist). Zur Verdeutlichung ist auch die Form der Aktuatorvorrichtung 70 in Figur 5C dargestellt. Diese umfasst insgesamt fünf Verschlie-βelemente 71, die mit den entsprechenden fünf Ventilsitzen die Ventile 321, 322, 323, 324 und 325 bilden.

Wie aus der gezeigten Anordnung ersichtlich, sind die Ventile 321 ... 325 zueinander in Serie und parallel zur Beobachtungskammer 50 angeordnet. Diese Anordnung kann dazu verwendet werden, die Beobachtungskammer 50 durch Schließen der Ventile 324 und 325 vom Kanalsystem 20 abzusperren, während das Fluid aus dem Kanalsystem 20 gespült wird.

Zur Veranschaulichung dieses Zusammenhangs wird auf den schematischen Schaltplan der Ventilanordnung in Figur 5B verwiesen. Wie darin gezeigt, erlaubt eine Öffnung des Bypass-Ventils 323 eine direkte Verbindung zwischen dem zweiten Flüssigkeitsreservoir 62 und dem dritten Flüssigkeitsreservoir. Wird nun an einem der Flüssigkeitsreservoire 62, 63 Luft oder ein anderes Gas (z.B. Argon) eingeleitet, so wird eine Flüssigkeit aus dem Kanalsystem 20 verdrängt. Dieser Prozess hat gleichzeitig keinen Einfluss auf die Beobachtungskammer 50, weil sie fluidisch von dem Kanalsystem 20 abtrennbar ist, indem die Ventile 324 und 325 geschlossen werden. Anschließend kann eine neue Flüssigkeit über die Flüssigkeitsreservoire 62, 63 eingeleitet werden. Dieser Medienwechsel erfolgt insbesondere ohne unnötige Verschwendung des neuen Fluids, weil das alte Fluid durch Luft praktisch vollständig verdrängt wurde und das neue Fluid deshalb direkt eingesetzt werden kann.

Besonders effektiv ist das Ausspülen einer Flüssigkeit aus dem Kanalsystem mit Hilfe eines Gases (z.B. Luft, Argon etc.) Dadurch wird die Flüssigkeit praktisch rückstandsfrei und unter Verwendung eines geringen Gasvolumens aus dem Kanalsystem entfernt und danach kann eine neue Flüssigkeit eingeleitet werden.

Die Mikrofluidsysteme der Figuren 4 und 5 können prinzipiell auch eine universelle Aktuatorvorrichtung umfassen, die für die Verwendung mit beiden zugehörigen Mikrofluidvorrichtungen ausgebildet ist. Diese universelle Aktuatorvorrichtung umfasst beispielweise fünf Verschließelemente, die mit den fünf Ventilsitzen 31 - 35 der Ausführungsform aus Figur 5 zusammenwirken können. Die Ausführungsform gemäß Figur 4 umfasst lediglich drei Ventilsitze (Ventile) 31, 32, 33, die sich an der gleichen Position befinden wie die zugehörigen Ventilsitze in der Ausführungsform gemäß Figur 5. Daher kann die Aktuatorvorrichtung der Ausführungsform gemäß Figur 5 auch für die Ausführungsform gemäß Figur 4 verwendet werden, wenn lediglich jene Verschlie-βelemente verwendet werden, die mit den drei Ventilsitzen 31, 32, 33 zusammenwirken.

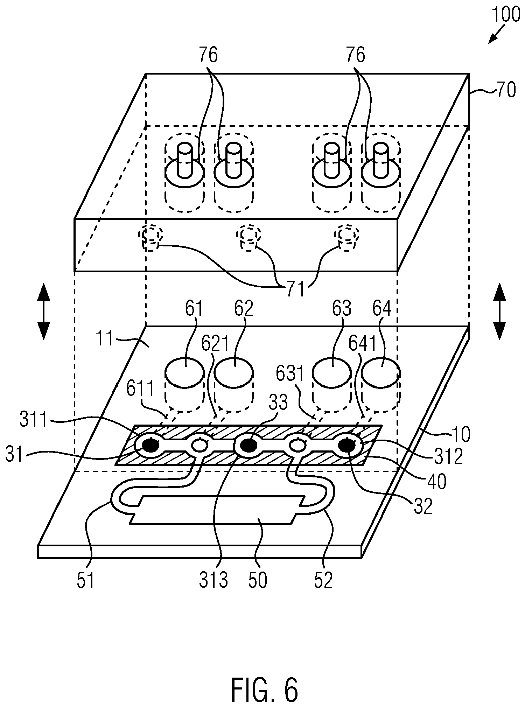

Das Mikrofluidsystem 100 gemäß einer weiteren Ausführungsform in Figur 6 basiert im Wesentlichen auf der Ausführungsform gemäß Figur 5 und umfasst die entsprechende Ventilanordnung bzw. Anordnung von Ventilsitzen im Substrat. Diese wird daher nicht erneut im Detail beschrieben.

Zunächst umfasst das Mikrofluidsystem 100 eine im Substrat 11 der Mikrofluidvorrichtung 10 integrierte Beobachtungskammer 50, die in Form eines Hohlraums im Substrat 11 ausgebildet und über einen Zufluss 51 und einen Abfluss 52 mit dem Kanalsystem 20 verbunden ist. Dabei sind der Zufluss 51 und Abfluss 52 selbst in Form eines Kanals im Substrat 11 ausgebildet. Alternativ kann die Beobachtungskammer 50, ebenso wie das Kanalsystem 20, in Form eines Grabens in der Oberfläche des Substrats 11 ausgebildet sein. Dieser Graben wird von der Membran 40, Klebefolie oder einem anderen Element fluidisch abgedichtet, sodass sich die Beobachtungskammer 50 ergibt.

Weiterhin sind die vier Flüssigkeitsreservoire 61 - 64 als Hohlräume im Substrat 11 der Mikrofluidvorrichtung 10 ausgebildet. Die einzelnen Flüssigkeitsreservoire sind über zugehörige Kanäle 611, 621, 631, 641 mit der Ventilanordnung und/oder dem Kanalsystem 20 verbunden. Der Kanal 611 verbindet das Flüssigkeitsreservoir 61 mit dem Ventilsitz 31, der Kanal 621 verbindet das Flüssigkeitsreservoir 62 mit dem Kanalsystem 20, der Kanal 631 verbindet das Flüssigkeitsreservoir 63 mit dem Kanalsystem 20 und der Kanal 641 verbindet das Flüssigkeitsreservoir 64 mit dem Ventilsitz 32. Der Ventilsitz 33 bildet den Teil eines Bypass-Ventils, wie es bereits in Figur 4A beschrieben wurde. Insgesamt realisiert das hier beschriebene Mikrofluidsystem 100 die in Figur 5B gezeigten Verbindungen der Flüssigkeitsreservoire, der Ventilsitze, des Kanalsystems und der Beobachtungskammer.

Die Aktuatorvorrichtung 70 umfasst drei Verschließelemente 71, die mit den Ventilsitzen 31, 32, 33 zusammenwirken, um die Ventile 311, 312, 313 zu bilden. Zudem umfasst die Aktuatorvorrichtung 70 insgesamt vier Pumpeinheiten 76. Diese sind in der gezeigten Ausführungsform als Kolben gezeigt, sind aber nicht auf diese Form beschränkt. Die Pumpeinheiten 76 sind ausgebildet, die vier Flüssigkeitsreservoire 61 - 64 mit Druck zu beaufschlagen und so die Flüssigkeit zu fördern. Weiterhin können die Pumpeinheiten die Funktion aufweisen, die jeweiligen Reservoire zu entlüften. Die Aktuatorvorrichtung 70 wird mit der Mikrofluidvorrichtung 10 verbunden und bildet so das Mikrofluidsystem 100. Als Verbindungsarten kommen beispielsweise eine Steckverbindung oder eine Klemmverbindung in Betracht. Ein weiterer Unterschied zum Ausführungsbeispiel gemäß Figur 5A-C besteht darin, dass die Mikrofluidvorrichtung 10 und die Aktuatorvorrichtung 70 nicht die gleichen Abmessungen in Länge und Breite (also ihrer Querschnittsfläche) aufweisen, damit die Beobachtungskammer 50 und die Aktuatorvorrichtung 70 nicht miteinander überlappen, wenn das Mikrofluidsystem 100 zusammengebaut ist. Anders gesagt ragt die Mikrofluidvorrichtung 10 über die Aktuatorvorrichtung 70 hinaus. Dies hat den Vorteil, dass es möglich sein soll, Mikroskopie an der Beobachtungskammer 50 durchzuführen, indem ein ausreichender optischer Zugang zur Verfügung steht. In der Regel befinden sich Zellen, Zellaggregate o.Ä. in der Beobachtungskammer, die mikroskopisch untersucht werden sollen. Dazu muss ein Objektiv (nicht gezeigt) von der ersten Seite oder der zweiten Seite des Substrats 11 möglichst nah an die Zellen herangebracht werden. Um diesen nötigen Freiraum für das Objektiv zu schaffen, stellt die beschriebene Konfiguration eine einfache Möglichkeit dar.

Zum Zweck der besagten mikroskopischen Untersuchungen kann das Substrat 11 im Bereich der Beobachtungskammer 50 transparent sein, insbesondere im sichtbaren Wellenlängenbereich. Sollte die Beobachtungskammer 50 durch die Membran 40 abgedichtet werden, gilt diese Anforderung auch für die Membran 40. In den übrigen Bereichen der Mikrofluidvorrichtung gelten an das Substrat 11 keine hohen optischen Anforderungen. Hier kann das Substrat 11 daher auch lediglich transluzent oder sogar opak sein.

Dieser Aufbau kommt vollständig ohne Flüssigkeitsschläuche aus, weil alle notwendigen Verbindungen zwischen den Elementen (insbesondere Flüssigkeitsreservoire, Kanalsystem und Beobachtungskammer) in Form von Kanälen im Substrat vorhanden sind. Dadurch wird die Komplexität des Aufbaus erheblich verringert. Darüber hinaus wird durch die Reduzierung der Schlauchverbindungen auch das Risiko von Kontamination und Blasenbildung verringert.

Es versteht sich, dass eine in das Substrat integrierte Beobachtungskammer und/oder die im Substrat integrierten Flüssigkeitsreservoire auch mit dem Ausführungsbeispiel gemäß Figur 5 kombinierbar ist/sind. Die Beobachtungskammer kann also auch in dem Substrat integriert sein, wenn zwischen dem zweiten Flüssigkeitsreservoir und der Beobachtungskammer, sowie zwischen dem dritten Flüssigkeitsreservoir und der Beobachtungskammer jeweils ein Ventil bzw. Ventilsitz vorgesehen ist. Auch in diesem Fall sollten die Aktuatorvorrichtung und die Beobachtungskammer nicht überlappen, sodass Mikroskopie an Zellen in der Beobachtungskammer möglich ist. Denkbar ist auch eine Mikrofluidvorrichtung wie in Figur 1, bei der die Beobachtungskammer im Substrat ausgebildet ist. Gegebenenfalls kann die Aktuatorvorrichtung ebenfalls die beschriebenen Pumpeinheiten umfassen.

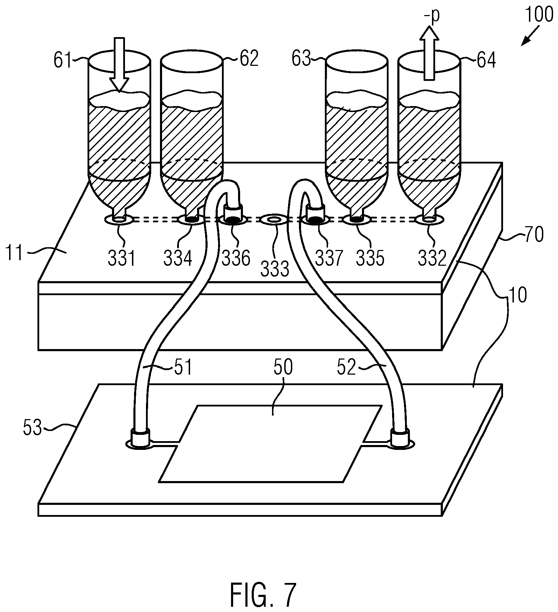

Figur 7 zeigt eine weitere Ausführungsform des Mikrofluidsystems sowie ein mögliches Anwendungsbeispiel. Wie auch das Ausführungsbeispiel gemäß Figur 5A umfasst das Mikrofluidsystem 100 vier Flüssigkeitsreservoire 61, 62, 63, 64, die direkt auf fluidische Anschlüsse der Mikrofluidvorrichtung 10 gesteckt sind. Dabei sind die Flüssigkeitsreservoir 61, 62, 63 zur Atmosphäre hin geöffnet und das Flüssigkeitsreservoir 64 ist an eine Pumpe (nicht gezeigt) angeschlossen, die einen Unterdruck -p erzeugen kann.

Im Substrat 11 der Mikrofluidvorrichtung sind insgesamt sieben Ventilsitze ausgebildet, die zusammen mit den zugehörigen Verschließelementen der Aktuatorvorrichtung 70 die Ventile 331 - 337 bilden. Die Mikrofluidvorrichtung umfasst weiterhin eine Beobachtungskammer 50 wie sie bereits im Ausführungsbeispiel der Figur 4A beschrieben ist. Auf weitergehende Erläuterungen wird deshalb an dieser Stelle verzichtet.

Mit dieser Anordnung ist es möglich, Flüssigkeit vom Reservoir 61 durch das Kanalsystem 20 in das Flüssigkeitsreservoir zu "ziehen". Dazu werden die sieben Ventile in die gezeigte Konfiguration gebracht, wobei ein schwarzer Vollkreis ein geschlossenes Ventil und ein weißer Vollkreis ein offenes Ventil anzeigt. Die Ventile 331, 332 und 333 sind also geöffnet, während die Ventile 334, 335, 336 und 337 geschlossen sind. Die Flüssigkeit strömt in Folge des am Flüssigkeitsreservoir 64 angelegten Unterdrucks aus dem Flüssigkeitsreservoir 61 durch das Kanalsystem 20, an der Beobachtungskammer 50 vorbei, über das Bypass-Ventil 333 zum Flüssigkeitsreservoir 64. Folglich muss nur an einem Flüssigkeitsreservoir ein Druck angelegt werden. Über die Flussrichtung im Kanalsystem 20 entscheidet allein die Stellung der Ventile (geöffnet oder geschlossen)

Anhand dieser Variante zeigt sich der genannte Vorteil, dass das Mikrofluidsystem mit einem Minimum an Schlauchverbindungen auskommen kann und gleichzeitig einen großen Umfang an Verwendungsmöglichkeiten bietet. Dadurch ist das vorliegende Mikrofluidsystem gegenüber bestehenden Lösungen wesentlich einfacher aufgebaut, reduziert ungünstige Totvolumina und verringert die Wahrscheinlichkeit für die Entstehung von unerwünschten Luftblasen.

Ein gemeinsames Prinzip des beschriebenen Mikrofluidsystems, insbesondere der Ausführungsbeispiele gemäß den Figuren 4 bis 7, ist das sogenannte "Common Rail Principle" (CRP). Dabei besteht eine Trennung der Beobachtungskammer von dem Kanalsystem durch zwei Ventile (zwischen Kanalsystem und Zufluss, sowie zwischen Kanalsystem und Abfluss). Das Kanalsystem ist mit einer Mehrzahl an Anschlüssen und damit verbundenen Flüssigkeitsreservoiren verbunden.

Das CRP bietet eine Reihe von Vorteilen. So kann das Mikrofluidsystem präpariert werden, indem das Kanalsystem unter Verwendung geringer Volumina mit einem bestimmten Fluid gefüllt wird. Experimente an biologischen Proben können beginnen, sobald das Fluid in die Beobachtungskammer gelangt. Da die Flüssigkeitsreservoire direkt auf die Anschlüsse der Mikrofluidvorrichtung gesteckt werden können, entsteht nur ein sehr geringes Totvolumen und es wird eine geringere Menge an Fluid tatsächlich benötigt. Ein weiterer Aspekt ist, wie bereits zuvor beschrieben, dass das Kanalsystem gespült werden kann, ohne die Beobachtungskammer zu beeinflussen. Dies ist besonders effizient, wenn eine Flüssigkeit mittels Luft aus dem Kanal gespült wird, weil die Flüssigkeit durch das Spülen mit Luft praktisch rückstandsfrei verdrängt wird.

## Patentansprüche

1. Mikrofluidvorrichtung (10), umfassend:
ein Substrat (11),
ein in dem Substrat angeordnetes Kanalsystem (20),
einen ersten und einen zweiten fluidischen Anschluss (12, 13) für die Zuführung eines Fluids in das Kanalsystem (20), wobei die fluidischen Anschlüsse (12, 13) an einer ersten Seite (11a) des Substrats (11) angeordnet sind,
einen ersten und einen zweiten Ventilsitz (31, 32), die beide in einer zweiten Seite (11b) des Substrats (11), die der ersten Seite (11a) gegenüberliegt, ausgebildet sind, und
eine elastische Membran (40), die zumindest einen Teil der zweiten Seite (11b) des Substrats (11) einschließlich der Ventilsitze (31, 32) bedeckt,
wobei der erste Ventilsitz (31) derart angeordnet ist, dass das Fluid vom ersten Anschluss (12) durch den ersten Ventilsitz (31) in das Kanalsystem (20) strömen kann, und
wobei der zweite Ventilsitz (32) derart angeordnet ist, dass das Fluid vom zweiten Anschluss (13) durch den zweiten Ventilsitz (32) in das Kanalsystem (20) strömen kann.

2. Mikrofluidvorrichtung (10) nach Anspruch 1, wobei das Kanalsystem (20) zumindest teilweise in Form eines Grabens an der zweiten Seite (11b) des Substrats (11) ausgebildet ist, und
wobei die Membran (40) den Graben fluidisch abdichtet.

3. Mikrofluidvorrichtung (10) nach einem der vorangegangenen Ansprüche, wobei die Membran (40) eine Silikonmembran, insbesondere umfassend Elastosil ist, und/oder wobei die Membran (40) eine Dicke im Bereich zwischen 5 µm und 5 mm, insbesondere zwischen 50 µm und 250 µm aufweist.

4. Mikrofluidvorrichtung (10) nach einem der vorangegangenen Ansprüche, wobei zwischen dem Substrat (11) und der Membran (40) weiterhin eine doppelseitige Klebefolie (41) angeordnet ist,
wobei die Klebefolie (41) zumindest die Ventilsitze (31, 32) ausspart, und
wobei die Membran (40) mittels der Klebefolie (41) an dem Substrat (11) angebracht ist.

5. Mikrofluidvorrichtung (10) nach einem der vorangegangenen Ansprüche, weiterhin umfassend eine Beobachtungskammer (50) die mit dem Kanalsystem (20) über einen Zulauf (51) und einen Ablauf (52) fluidisch verbunden ist,
wobei die Beobachtungskammer (50) insbesondere in dem Substrat (11) ausgebildet ist.

6. Mikrofluidvorrichtung (10) nach Anspruch 5, wobei zwischen dem Zulauf (51) der Beobachtungskammer (50) und dem Kanalsystem (20), sowie zwischen dem Ablauf (52) der Beobachtungskammer (50) und dem Kanalsystem (20) jeweils ein weiterer Ventilsitz (34, 35) angeordnet ist, und
wobei das Kanalsystem (20) insbesondere einen Bypass umfasst, sodass das Fluid durch das Kanalsystem (20) an der Beobachtungskammer (50) vorbeiströmen kann.

7. Mikrofluidvorrichtung (10) nach einem der vorangegangenen Ansprüche, weiterhin umfassend ein erstes Flüssigkeitsreservoir (61) und ein zweites Flüssigkeitsreservoir (62), wobei das erste Flüssigkeitsreservoir (61) mit dem ersten fluidischen Anschluss (12) verbunden ist,
wobei das zweite Flüssigkeitsreservoir (62) mit dem zweiten fluidischen Anschluss (13) verbunden ist, und
wobei das erste Flüssigkeitsreservoir (61) und/oder das zweite Flüssigkeitsreservoir (62) insbesondere direkt auf den jeweiligen Anschluss (12, 13) gesteckt ist/sind.

8. Mikrofluidvorrichtung (10) nach Anspruch 7, weiterhin umfassend:
einen dritten und einen vierten fluidischen Anschluss (14, 15), und
ein drittes und ein viertes Flüssigkeitsreservoir (63, 64), das mit dem dritten bzw. vierten fluidischen Anschluss (14, 15) verbunden ist, und
wobei der dritte und der vierte Anschluss (14, 15) mit dem Kanalsystem (20) fluidisch verbunden sind.

9. Mikrofluidvorrichtung (10) nach Anspruch 8, wobei zwischen dem dritten fluidischen Anschluss (14) und der Beobachtungskammer (50), sowie zwischen dem vierten fluidischen Anschluss (15) und der Beobachtungskammer (50) jeweils ein Ventilsitz (34, 35) angeordnet ist, und
wobei insbesondere die Ventilsitze in Serie entlang des Kanalsystems (20) angeordnet sind und die Beobachtungskammer (50) parallel zu den in Serie geschalteten Ventilsitzen angeordnet ist.

10. Mikrofluidsystem (100), umfassend:
eine Mikrofluidvorrichtung (10) nach einem der vorangegangenen Ansprüche, und eine Aktuatorvorrichtung (70),
wobei die Mikrofluidvorrichtung (10) und die Aktuatorvorrichtung (70) zerstörungsfrei lösbar derart miteinander verbindbar sind, dass die Membran (40) zwischen dem Substrat (11) und der Aktuatorvorrichtung (70) angeordnet ist,
wobei die Aktuatorvorrichtung (70) ausgebildet ist, eine Kraft auf die Membran (40) auszuüben,
wobei der erste Ventilsitz (31), die Membran (40) und die Aktuatorvorrichtung (70) ein erstes Ventil (311; 321) bilden,
wobei der zweite Ventilsitz (32), die Membran (40) und die Aktuatorvorrichtung (70) ein zweites Ventil (312; 322) bilden, und
wobei die Aktuatorvorrichtung (70) mit der Mikrofluidvorrichtung (10) derart zusammenwirkt, dass das erste (311; 321) und zweite Ventil (312; 322) eine geschlossene Position und eine geöffnete Position aufweisen, wobei in der geschlossenen Position die Membran (40) durch die Kraft in den jeweiligen Ventilsitz (31, 32) gedrückt wird, sodass das zugehörige Ventil in die geschlossene Position bringbar ist.

11. Mikrofluidsystem (100) nach Anspruch 10, wobei die Aktuatorvorrichtung (70) ebenso viele Verschließelemente (71) umfasst wie die Mikrofluidvorrichtung (10) Ventilsitze aufweist,
wobei jedes der Verschließelemente (71) ein Kolben oder Stößel ist, und
wobei jedes der Ventile durch einen der Ventilsitze, das zugehörige Verschließelement (71) und die Membran (40) gebildet wird.

12. Mikrofluidsystem (100) nach Anspruch 11, wobei eine der Membran (40) zugewandte Seite jedes Kolbens oder Stößels abgerundet ist, und
wobei jeder der Ventilsitze insbesondere angefast ist.

13. Mikrofluidsystem (100) nach Anspruch 11 oder 12, wobei die Aktuatorvorrichtung (70) eine Antriebseinheit (75) umfasst, die ausgebildet ist, die Verschließelemente (71) elektromagnetisch, hydraulisch, elektromechanisch und/oder pneumatisch anzutreiben.

14. Mikrofluidsystem (100) nach einem Ansprüche 10 bis 13, wobei die Mikrofluidvorrichtung (10) eine größere Fläche als die Aktuatorvorrichtung (70) aufweist, sodass die Mikrofluidvorrichtung (10) über die Aktuatorvorrichtung (70) hinausragt, wenn die Mikrofluidvorrichtung (10) und die Aktuatorvorrichtung (70) miteinander verbunden sind,
wobei die Beobachtungskammer (50) im Substrat (11) der Mikrofluidvorrichtung (10) ausgebildet ist, und
wobei sich die Beobachtungskammer (50) in dem Teil der Mikrofluidvorrichtung (10) befindet, der über die Aktuatorvorrichtung (70) hinausragt.

15. Mikrofluidsystem (100) nach einem der Ansprüche 10 bis 14, wobei die Mikrofluidvorrichtung (10) ein Wegwerfartikel für den Einmalgebrauch ist, und/oder wobei die Aktuatorvorrichtung (70) wiederverwendbar ist.

## Geänderte Patentansprüche

### Geänderte Patentansprüche gemäss Regel 137(2) EPÜ.

1. Mikrofluidvorrichtung (10), umfassend:
ein Substrat (11),
ein in dem Substrat angeordnetes Kanalsystem (20),
einen ersten und einen zweiten fluidischen Anschluss (12, 13) für die Zuführung eines Fluids in das Kanalsystem (20), wobei die fluidischen Anschlüsse (12, 13) an einer ersten Seite (11a) des Substrats (11) angeordnet sind,
einen ersten und einen zweiten Ventilsitz (31, 32), die beide in einer zweiten Seite (11b) des Substrats (11), die der ersten Seite (11a) gegenüberliegt, ausgebildet sind,
eine elastische Membran (40), die zumindest einen Teil der zweiten Seite (11b) des Substrats (11) einschließlich der Ventilsitze (31, 32) bedeckt, und
eine Beobachtungskammer (50) die mit dem Kanalsystem (20) über einen Zulauf (51) und einen Ablauf (52) fluidisch verbunden ist,
wobei der erste Ventilsitz (31) derart angeordnet ist, dass das Fluid vom ersten Anschluss (12) durch den ersten Ventilsitz (31) in das Kanalsystem (20) strömen kann,
wobei der zweite Ventilsitz (32) derart angeordnet ist, dass das Fluid vom zweiten Anschluss (13) durch den zweiten Ventilsitz (32) in das Kanalsystem (20) strömen kann,
wobei zwischen dem Zulauf (51) der Beobachtungskammer (50) und dem Kanalsystem (20), sowie zwischen dem Ablauf (52) der Beobachtungskammer (50) und dem Kanalsystem (20) jeweils ein weiterer Ventilsitz (34, 35) angeordnet ist, und
wobei das Kanalsystem (20) einen Bypass umfasst, sodass das Fluid durch das Kanalsystem (20) an der Beobachtungskammer (50) vorbeiströmen kann.

2. Mikrofluidvorrichtung (10) nach Anspruch 1, wobei das Kanalsystem (20) zumindest teilweise in Form eines Grabens an der zweiten Seite (11b) des Substrats (11) ausgebildet ist, und
wobei die Membran (40) den Graben fluidisch abdichtet.

3. Mikrofluidvorrichtung (10) nach einem der vorangegangenen Ansprüche, wobei die Membran (40) eine Silikonmembran, insbesondere umfassend Elastosil ist, und/oder wobei die Membran (40) eine Dicke im Bereich zwischen 5 µm und 5 mm, insbesondere zwischen 50 µm und 250 µm aufweist.

4. Mikrofluidvorrichtung (10) nach einem der vorangegangenen Ansprüche, wobei zwischen dem Substrat (11) und der Membran (40) weiterhin eine doppelseitige Klebefolie (41) angeordnet ist,
wobei die Klebefolie (41) zumindest die Ventilsitze (31, 32) ausspart, und
wobei die Membran (40) mittels der Klebefolie (41) an dem Substrat (11) angebracht ist.

5. Mikrofluidvorrichtung (10) nach einem der vorangegangenen Ansprüche,
wobei die Beobachtungskammer (50) in dem Substrat (11) ausgebildet ist.

6. Mikrofluidvorrichtung (10) nach einem der vorangegangenen Ansprüche, weiterhin umfassend ein erstes Flüssigkeitsreservoir (61) und ein zweites Flüssigkeitsreservoir (62), wobei das erste Flüssigkeitsreservoir (61) mit dem ersten fluidischen Anschluss (12) verbunden ist,
wobei das zweite Flüssigkeitsreservoir (62) mit dem zweiten fluidischen Anschluss (13) verbunden ist, und
wobei das erste Flüssigkeitsreservoir (61) und/oder das zweite Flüssigkeitsreservoir (62) insbesondere direkt auf den jeweiligen Anschluss (12, 13) gesteckt ist/sind.

7. Mikrofluidvorrichtung (10) nach Anspruch 6, weiterhin umfassend:
einen dritten und einen vierten fluidischen Anschluss (14, 15), und
ein drittes und ein viertes Flüssigkeitsreservoir (63, 64), das mit dem dritten bzw. vierten fluidischen Anschluss (14, 15) verbunden ist, und
wobei der dritte und der vierte Anschluss (14, 15) mit dem Kanalsystem (20) fluidisch verbunden sind.

8. Mikrofluidvorrichtung (10) nach Anspruch 7, wobei zwischen dem dritten fluidischen Anschluss (14) und der Beobachtungskammer (50), sowie zwischen dem vierten fluidischen Anschluss (15) und der Beobachtungskammer (50) jeweils ein Ventilsitz (34, 35) angeordnet ist, und
wobei insbesondere die Ventilsitze in Serie entlang des Kanalsystems (20) angeordnet sind und die Beobachtungskammer (50) parallel zu den in Serie geschalteten Ventilsitzen angeordnet ist.

9. Mikrofluidsystem (100), umfassend:
eine Mikrofluidvorrichtung (10) nach einem der vorangegangenen Ansprüche, und
eine Aktuatorvorrichtung (70),
wobei die Mikrofluidvorrichtung (10) und die Aktuatorvorrichtung (70) zerstörungsfrei lösbar derart miteinander verbindbar sind, dass die Membran (40) zwischen dem Substrat (11) und der Aktuatorvorrichtung (70) angeordnet ist,
wobei die Aktuatorvorrichtung (70) ausgebildet ist, eine Kraft auf die Membran (40) auszuüben,
wobei der erste Ventilsitz (31), die Membran (40) und die Aktuatorvorrichtung (70) ein erstes Ventil (311; 321) bilden,
wobei der zweite Ventilsitz (32), die Membran (40) und die Aktuatorvorrichtung (70) ein zweites Ventil (312; 322) bilden, und
wobei die Aktuatorvorrichtung (70) mit der Mikrofluidvorrichtung (10) derart zusammenwirkt, dass das erste (311; 321) und zweite Ventil (312; 322) eine geschlossene Position und eine geöffnete Position aufweisen, wobei in der geschlossenen Position die Membran (40) durch die Kraft in den jeweiligen Ventilsitz (31, 32) gedrückt wird, sodass das zugehörige Ventil in die geschlossene Position bringbar ist.

10. Mikrofluidsystem (100) nach Anspruch 9, wobei die Aktuatorvorrichtung (70) ebenso viele Verschließelemente (71) umfasst wie die Mikrofluidvorrichtung (10) Ventilsitze aufweist, wobei jedes der Verschließelemente (71) ein Kolben oder Stößel ist, und
wobei jedes der Ventile durch einen der Ventilsitze, das zugehörige Verschließelement (71) und die Membran (40) gebildet wird.

11. Mikrofluidsystem (100) nach Anspruch 10, wobei eine der Membran (40) zugewandte Seite jedes Kolbens oder Stößels abgerundet ist, und
wobei jeder der Ventilsitze insbesondere angefast ist.

12. Mikrofluidsystem (100) nach Anspruch 10 oder 11, wobei die Aktuatorvorrichtung (70) eine Antriebseinheit (75) umfasst, die ausgebildet ist, die Verschließelemente (71) elektromagnetisch, hydraulisch, elektromechanisch und/oder pneumatisch anzutreiben.

13. Mikrofluidsystem (100) nach einem Ansprüche 9 bis 12, wobei die Mikrofluidvorrichtung (10) eine größere Fläche als die Aktuatorvorrichtung (70) aufweist, sodass die Mikrofluidvorrichtung (10) über die Aktuatorvorrichtung (70) hinausragt, wenn die Mikrofluidvorrichtung (10) und die Aktuatorvorrichtung (70) miteinander verbunden sind,
wobei die Beobachtungskammer (50) im Substrat (11) der Mikrofluidvorrichtung (10) ausgebildet ist, und
wobei sich die Beobachtungskammer (50) in dem Teil der Mikrofluidvorrichtung (10) befindet, der über die Aktuatorvorrichtung (70) hinausragt.

14. Mikrofluidsystem (100) nach einem der Ansprüche 9 bis 13, wobei die Mikrofluidvorrichtung (10) ein Wegwerfartikel für den Einmalgebrauch ist, und/oder
wobei die Aktuatorvorrichtung (70) wiederverwendbar ist.
